(19) Europäisches Patentamt
European Patent Office
Office européen des brevets

(11) **EP 3 010 572 B1**

(12) **EUROPÄISCHE PATENTSCHRIFT**

(45) Veröffentlichungstag und Bekanntmachung des Hinweises auf die Patenterteilung:
**20.03.2019 Patentblatt 2019/12**

(51) Int Cl.:
***A61M 21/00*** *(2006.01)*

(21) Anmeldenummer: **14747020.7**

(86) Internationale Anmeldenummer:
**PCT/EP2014/066611**

(22) Anmeldetag: **01.08.2014**

(87) Internationale Veröffentlichungsnummer:
**WO 2015/018755 (12.02.2015 Gazette 2015/06)**

(54) **VORRICHTUNG ZUR EICHUNG EINER AKUSTISCHEN DESYNCHRONISIERENDEN NEUROSTIMULATION**

APPARATUS FOR CALIBRATING ACOUSTIC DESYNCHRONIZING NEUROSTIMULATION

DISPOSITIF D'ÉTALONNAGE D'UNE NEUROSTIMULATION ACOUSTIQUE DÉSYNCHRONISANTE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **08.08.2013 DE 102013013278**

(43) Veröffentlichungstag der Anmeldung:
**27.04.2016 Patentblatt 2016/17**

(73) Patentinhaber: **Forschungszentrum Jülich GmbH 52425 Jülich (DE)**

(72) Erfinder: **TASS, Peter Alexander 52428 Jülich (DE)**

(74) Vertreter: **Müller-Boré & Partner Patentanwälte PartG mbB Friedenheimer Brücke 21 80639 München (DE)**

(56) Entgegenhaltungen:
**DE-A1-102010 016 461    US-A- 4 883 067 US-A1- 2005 113 871**

- **BORYS LYSYANSKY ET AL: "Desynchronizing anti-resonance effect of m: n ONOFF coordinated reset stimulation;Desynchronizing anti-resonance effect of the ON-OFF CR stimulation", JOURNAL OF NEURAL ENGINEERING, INSTITUTE OF PHYSICS PUBLISHING, BRISTOL, GB, Bd. 8, Nr. 3, 10. Mai 2011 (2011-05-10), Seite 36019, XP020205979, ISSN: 1741-2552, DOI: 10.1088/1741-2560/8/3/036019**

**Beschreibung**

Die Erfindung bezieht sich auf eine Vorrichtung zur Eichung einer akustischen desynchronisierenden Neurostimulation.

Bei Patienten mit Hirnerkrankungen, z.B. Tinnitus, Depression, Zwangserkrankungen, ADHS, Schizophrenie, sind Nervenzellverbände in umschriebenen Bereichen des Gehirns krankhaft, z.B. übersteigert synchron, aktiv. In diesem Fall bildet eine große Anzahl von Neuronen synchron Aktionspotentiale aus; die beteiligten Neuronen feuern übermäßig synchron. Beim Gesunden hingegen feuern die Neuronen in diesen Hirngebieten qualitativ anders, z.B. auf unkorrelierte Weise.

Zur Behandlung des chronischen subjektiven Tinnitus wurde die akustische Coordinated Reset (CR)-Stimulation entwickelt, welche gezielt krankhaft synchroner neuronaler Aktivität entgegenwirkt (P.A. Tass, I. Adamchic, H.-J. Freund, T. von Stackelberg, C.Hauptmann: Counteracting tinnitus by acoustic coordinated reset neuromodulation. Restorative Neurology and Neuroscience 30, 137-159 (2012); I. Adamchic, T. Toth, C. Hauptmann, P.A. Tass: Reversing pathologically increased EEG power by acoustic CR neuromodulation. Human Brain Mapping (in press)) und auch die krankhaft veränderten Interaktionen (sog. effektive Konnektivität) zwischen verschiedenen Hirnarealen zu normalisieren vermag (A.N. Silchenko, I. Adamchic, C. Hauptmann, P.A. Tass: Impact of acoustic coordinated reset neuromodulation on effective connectivity in a neural network of phantom sound. Neuroimage 77, 133-147 (2013)). Die akustische CR-Stimulation zeichnet sich durch therapeutische Wirksamkeit und Sicherheit aus (P.A. Tass, I. Adamchic, H.-J. Freund, T. von Stackelberg, C.Hauptmann: Counteracting tinnitus by acoustic coordinated reset neuromodulation. Restorative Neurology and Neuroscience 30, 137-159 (2012)).

Von zentraler Bedeutung für die Wirksamkeit der akustischen CR-Stimulation ist, dass die verschiedenen Stimulationsorte tatsächlich in der zu stimulierenden Neuronenpopulation liegen. Es gibt kein bildgebendes Verfahren, welches die räumliche Ausdehnung der krankhaften neuronalen Synchronisation ermitteln könnte. Die CR-Therapietöne werden somit nicht mittels eines objektiven Verfahrens, sondern mittels einer audiometrischen, auf Psychoakustik beruhenden und somit je nach Patienten in unterschiedlichem Ausmaß fehlerhaften Prozedur bestimmt. Bei der audiometrischen Anpassung der CR-Töne wird zuerst die dominante Tinnitusfrequenz audiometrisch ermittelt. Dies funktioniert typischerweise nicht bei Patienten mit rauschartigem Tinnitus, sondern nur bei Patienten mit tonalem Tinnitus. Die Tonhöhen der CR-Therapietöne sind starr vorgegeben (P.A. Tass, I. Adamchic, H.-J. Freund, T. von Stackelberg, C.Hauptmann: Counteracting tinnitus by acoustic coordinated reset neuromodulation. Restorative Neurology and Neuroscience 30, 137-159 (2012)). In einem zweiten Schritt wird die Lautstärke der Therapietöne mit starr vorgegebenen Tonhöhen (die z.B. im Bereich von 77% bis zu 140 % der dominanten Tonhöhe des Tinnitus liegen) abgeglichen (d.h. die Lautstärke der CR-Therapietöne wird subjektiv möglichst gleich laut eingestellt).

Ca. 25 % der Patienten mit tonalem Tinnitus sprechen auf diese Therapie nicht an (P.A. Tass, I. Adamchic, H.-J. Freund, T. von Stackelberg, C.Hauptmann: Counteracting tinnitus by acoustic coordinated reset neuromodulation. Restorative Neurology and Neuroscience 30, 137-159 (2012)). Dies entspricht ungefähr dem Prozentsatz der Patienten mit tonalem Tinnitus, welche keinen Tonhöhenabgleich zwischen ihrem Tinnitus und einem Computer-Vergleichston machen können. Außerdem ist die CR-Behandlung in ihrer derzeitigen Form (d.h. mit der oben geschilderten audiometrischer Anpassung der CR-Töne) für Patienten mit engbandigem rauschartigen Tinnitus bedingt und für Patienten mit breitbandigem rauschartigen Tinnitus nicht geeignet. Einerseits fehlt eine (audiometrisch bestimmbare) dominante Tinnitusfrequenz und andererseits ist die starr vorgegebene Anordnung der Tonhöhen der CR-Therapietöne (z.B. im Bereich von 77% bis zu 140 % der dominanten Tonhöhe des Tinnitus) typischerweise ungeeignet, da der dem rauschartigen Tinnitus zugrunde liegende krankhaft synchrone Herd im zentralen auditorischen System z.B. eine andere Ausdehnung aufweist als im Fall eines tonalen Tinnitus.

Eine analoge Situation ergibt sich im Falle der Behandlung anderer Hirnerkrankungen, wie z.B. ADHS, Depression, Zwangserkrankungen oder Schizophrenie, mit akustischer CR-Stimulation. Die Auswahl der geeigneten CR-Therapietöne konnte bis jetzt nur audiometrisch bzw. mit trial and error durchgeführt werden. Zeitaufwändiges Probieren garantiert die optimale Wirksamkeit der nicht-invasiven CR-Therapie nicht, da einerseits nicht alle möglichen Stimulationsorte im Gehirn systematisch erschlossen und getestet werden, und andererseits die Patienten durch lange Untersuchungen strapaziert werden, so dass die Mitarbeit der Patienten naturgemäß leidet, und die Ergebnisse der Testung schlechter werden.

Bisher wurden die Eichvorgänge durch audiometrische/psychophysische Anpassungen (z.B. bei der akustischen CR-Stimulation zur Behandlung des Tinnitus) vollzogen. Der Erfindung liegt die Aufgabe zugrunde, eine Vorrichtung anzugeben, mit der sich eine objektive und hinreichend schnell durchführbare Eichung durchführen lässt.

Das Dokument DE 10 2010 016 461 A1 offenbart eine Vorrichtung zur Stimulation von Neuronen mit einer krankhaft synchronen und oszillatorischen neuronalen Aktivität. Die Vorrichtung umfasst eine Stimulationseinheit zur Applikation von pulsförmigen Tönen an einen Patienten, wobei die Töne Neuronen des Patienten stimulieren, eine Messeinheit zum Aufnehmen von Messsignalen, die eine neuronale Aktivität der stimulierten Neuronen wiedergeben, und eine Steuer- und Analyseeinheit zur Steuerung der Stimulationseinheit und zur Analyse der Messsignale.

Die vorstehende Aufgabe wird dadurch gelöst, dass die subjektive Messung (z.B. audiometrische Anpassung bei akustischer CR-Stimulation) durch eine objektive, hinreichend schnell durchzuführende Untersuchung mit der erfindungsgemäßen Vorrichtung ersetzt wird. Letztere ermöglicht, eine elektrophysiologisch basierte und hinreichend schnell durchführbare Messung der Reizantworten des Gehirns zur Eichung der optimalen Stimulationsparameter und Stimulationsorte zu verwenden.

Die Aufgabe wird mit folgender erfindungsgemäßer Vorrichtung gelöst:
Die erfindungsgemäße Vorrichtung besteht aus (i) einer Messeinheit mit nicht-invasiven oder (weniger bevorzugt) invasiven Sensoren zur elektrophysiologischen Messung der pathologischen neuronalen Aktivität oder einer diese repräsentierenden bzw. mit ihr hinreichend eng korrelierenden Aktivität (z.B. Muskelaktivität), (ii) einer Stimulationseinheit zur Applikation von akustischen Stimuli und (iii) einer Steuer- und Analyseeinheit.

Die Erfindung ist in zwei alternativen Implementierungen durch die anhängenden Ansprüche 1 und 13 definiert und wird nachfolgend in beispielhafter Weise unter Bezugnahme auf die Zeichnungen näher erläutert.

Es kann durchaus vorgesehen sein, dass die einzelnen Komponenten der erfindungsgemäßen Vorrichtung, insbesondere die Messeinheit, die Stimulationseinheit und die Steuer- und Analyseeinheit baulich voneinander getrennt sind. Die erfindungsgemäße Vorrichtung kann daher auch als System aufgefasst werden.

Die erfindungsgemäße Vorrichtung kann insbesondere zur Behandlung von neurologischen oder psychiatrischen Erkrankungen eingesetzt werden, z.B. Morbus Parkinson, essentiellem Tremor, Tremor infolge von Multipler Sklerose sowie anderen pathologischen Tremores, Dystonie, Epilepsie, Depression, Bewegungsstörungen, Kleinhirnerkrankungen, Zwangserkrankungen, Tourette-Syndrom, Autismus, Funktionsstörungen nach Schlaganfall, Spastik, Tinnitus, Schlafstörungen, Schizophrenie, Reizdarm-Syndrom, Suchterkrankungen, Borderline-Persönlichkeitsstörung, Aufmerksamkeits-Defizit-Syndrom, Aufmerksamkeits-Defizit-Hyperaktivitäts-Syndrom, Spielsucht, Neurosen, Fresssucht, Magersucht, Essstörungen, Burnout-Syndrom, Fibromyalgie, Migräne, Cluster-Kopfschmerz, allgemeiner Kopfschmerz, Neuralgie, Ataxie, Tic-Störung oder Hypertonie, sowie weiteren Erkrankungen, die durch krankhaft gesteigerte neuronale Synchronisation gekennzeichnet sind.

Die vorstehend genannten Krankheiten können durch eine Störung der bioelektrischen Kommunikation von Neuronenverbänden, die in spezifischen Schaltkreisen zusammengeschlossen sind, verursacht werden. Hierbei generiert eine Neuronenpopulation anhaltend krankhafte neuronale Aktivität und möglicherweise eine damit verbundene krankhafte Konnektivität (Netzwerkstruktur). Dabei bilden eine große Anzahl von Neuronen synchron Aktionspotentiale aus, d. h., die beteiligten Neuronen feuern übermäßig synchron. Hinzu kommt, dass die kranke Neuronenpopulation eine oszillatorische neuronale Aktivität aufweist, d. h., die Neuronen feuern rhythmisch. Im Fall von neurologischen oder psychiatrischen Erkrankungen liegt die mittlere Frequenz der krankhaften rhythmischen Aktivität der betroffenen Neuronenverbände etwa im Bereich von 1 bis 30 Hz, kann aber auch außerhalb dieses Bereichs liegen. Bei gesunden Menschen feuern die Neuronen hingegen qualitativ anders, z.B. auf unkorrelierte Weise.

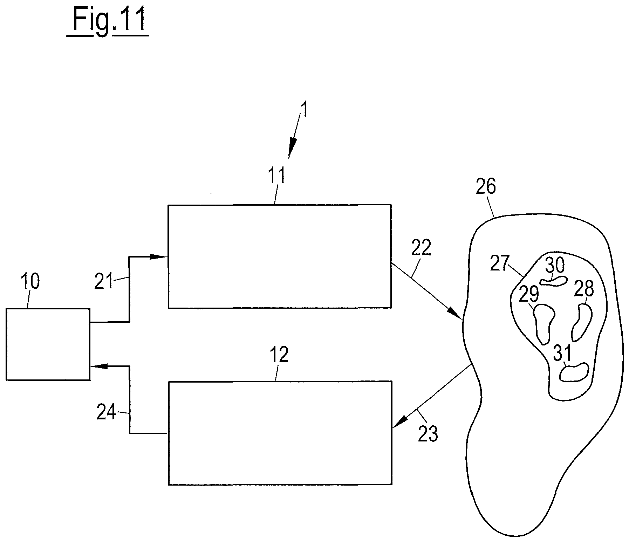

In Fig. 11 ist schematisch als Ausführungsbeispiel der Erfindung eine Vorrichtung 1 zur Eichung der Stimulationsparameter einer akustischen desynchronisierenden Neurostimulation dargestellt. Die Vorrichtung 1 besteht aus einer Steuer- und Analyseeinheit 10, einer Stimulationseinheit 11 und einer Messeinheit 12. Während des Betriebs der Vorrichtung 1 führt die Steuer- und Analyseeinheit 10 u. a. eine Steuerung der Stimulationseinheit 11 durch. Dazu erzeugt die Steuer- und Analyseeinheit 10 Steuersignale 21, die von der Stimulationseinheit 11 entgegengenommen werden. Die Stimulationseinheit 11 erzeugt anhand der Steuersignale 21 Reize 22, d. h. Töne, die einem Patienten verabreicht werden. Die Stimulationseinheit 11 und insbesondere auch die Steuer- und Analyseeinheit 10 sind nicht-invasive Einheiten, d.h., während des Betriebs der Vorrichtung 1 befinden sie sich außerhalb des Körpers des Patienten und werden nicht operativ in den Körper des Patienten implantiert.

Der durch die Töne 22 erzielte Stimulationseffekt wird mit Hilfe der Messeinheit 12 kontrolliert. Die Messeinheit 12 nimmt ein oder mehrere am Patienten gemessene Messsignale 23 auf, wandelt diese gegebenenfalls in elektrische Signale 24 um und führt diese der Steuer- und Analyseeinheit 10 zu. Insbesondere kann mittels der Messeinheit 12 die neuronale Aktivität in dem stimulierten Zielgebiet oder einem mit dem Zielgebiet verbundenen Gebiet gemessen werden, wobei die neuronale Aktivität dieses Gebiets mit der neuronalen Aktivität des Zielgebiets hinreichend eng korreliert (z.B. Muskelaktivität). Die Steuer- und Analyseeinheit 10 verarbeitet die Signale 24, z.B. können die Signale 24 verstärkt und gefiltert werden, und analysiert die verarbeiteten Signale 24. Anhand der Ergebnisse dieser Analyse steuert die Steuer- und Analyseeinheit 10 insbesondere die Stimulationseinheit 11 an. Zur Durchführung ihrer Aufgaben kann die Steuer- und Analyseeinheit 10 beispielsweise einen Prozessor (z.B. einen Mikrocontroller) enthalten.

In Fig. 11 ist die Vorrichtung 1 während einer CR-Stimulation dargestellt. Im Gehirn 26 des Patienten weist mindestens eine Neuronenpopulation 27 eine wie krankhaft synchrone und oszillatorische neuronale Aktivität auf. Die Stimulationseinheit 11 verabreicht dem Patienten die Töne 22 derart, die über die Ohren des Patienten aufgenommen werden und von dort über das Nervensystem an die krankhaft aktive Neuronenpopulation 27 im Gehirn 26 weitergeleitet werden. Die Töne 22 sind so ausgestaltet, dass die krankhaft synchrone Aktivität der Neuronenpopulation 27 desynchronisiert wird. Eine durch die Stimulation bewirkte Senkung der Koinzidenzrate der Neuronen kann zu einer Senkung der synaptischen Gewichte und somit zu einem Verlernen der Tendenz zur Produktion krankhaft synchroner Aktivität führen.

Akustische Reize werden im Innenohr in Nervenimpulse umgesetzt und über den Hörnerv zu dem auditorischen Cortex weitergeleitet. Durch die tonotope Anordnung des auditorischen Cortex wird bei der akustischen Stimulation des Innenohres mit einer bestimmten Frequenz ein bestimmter Teil des auditorischen Cortex aktiviert.

Die Stimulationseinheit 11 kann demnach unterschiedliche Bereiche des Gehirns 26 separat stimulieren, indem die applizierten Reize 22 über Nervenleitungen an unterschiedliche Zielgebiete, die im Gehirn 26 liegen, weitergeleitet werden. Die Zielgebiete können während der CR-Stimulation mit eventuell unterschiedlichen und/oder zeitversetzten Reizen 22 stimuliert werden.

Bei der CR-Stimulation werden der Neuronenpopulation 27, die eine krankhaft synchrone und oszillatorische Aktivität aufweist, Reize 22 verabreicht, welche in der Neuronenpopulation 27 ein Zurücksetzen, einen sogenannten Reset, der Phase der neuronalen Aktivität der stimulierten Neuronen bewirken. Durch das Zurücksetzen wird die Phase der stimulierten Neuronen unabhängig von dem aktuellen Phasenwert auf einen oder nahe zu einem bestimmten Phasenwert, z.B. 0°, gesetzt (in der Praxis ist es nicht möglich, einen bestimmten Phasenwert exakt einzustellen, dies ist für eine erfolgreiche CR-Stimulation aber auch nicht erforderlich). Somit wird die Phase der neuronalen Aktivität der krankhaften Neuronenpopulation 27 mittels einer gezielten Stimulation kontrolliert. Da es ferner möglich ist, die krankhafte Neuronenpopulation 27 an unterschiedlichen Stellen zu stimulieren, kann die Phase der neuronalen Aktivität der krankhaften Neuronenpopulation 27 an den unterschiedlichen Stimulationsstellen zu unterschiedlichen Zeitpunkten zurückgesetzt werden. Im Ergebnis wird dadurch die krankhafte Neuronenpopulation 27, deren Neuronen zuvor synchron und mit gleicher Frequenz und Phase aktiv waren, in mehrere Subpopulationen aufgespalten, die in Fig. 11 schematisch dargestellt sind und mit den Bezugszeichen 28, 29, 30 und 31 gekennzeichnet sind (beispielhaft sind hier vier Subpopulationen dargestellt). Innerhalb einer der Subpopulationen 28 bis 31 sind die Neuronen nach dem Zurücksetzen der Phase weiterhin synchron und feuern auch weiterhin mit derselben pathologischen Frequenz, aber jede der Subpopulationen 28 bis 31 weist bezüglich ihrer neuronalen Aktivität die Phase auf, die ihr durch den Stimulationsreiz aufgezwungen wurde. Dies bedeutet, dass die neuronalen Aktivitäten der einzelnen Subpopulationen 28 bis 31 nach dem Zurücksetzen ihrer Phasen weiterhin einen in etwa sinusförmigen Verlauf mit derselben pathologischen Frequenz haben, aber unterschiedliche Phasen.

Bedingt durch die krankhafte Interaktion zwischen den Neuronen ist der durch die Stimulation erzeugte Zustand mit mindestens zwei Subpopulationen instabil, und die gesamte Neuronenpopulation 27 nähert sich schnell einem Zustand kompletter Desynchronisation, in welchem die Neuronen unkorreliert feuern. Der gewünschte Zustand, d.h. die komplette Desynchronisation, ist somit nach der zeitversetzten (oder phasen-verschobenen) Applikation der phasenrücksetzenden Reize 22 nicht sofort vorhanden, sondern stellt sich meist innerhalb weniger Perioden oder gar in weniger als einer Periode der pathologischen Frequenz ein.

Eine Theorie zur Erklärung des Stimulationserfolgs basiert darauf, dass die letztlich gewünschte Desynchronisation durch die krankhaft gesteigerte Interaktion zwischen den Neuronen erst ermöglicht wird. Hierbei wird ein Selbstorganisationsprozess ausgenutzt, der für die krankhafte Synchronisation verantwortlich ist. Derselbe bewirkt, dass auf eine Aufteilung einer Gesamtpopulation 27 in Subpopulationen 28 bis 31 mit unterschiedlichen Phasen eine Desynchronisation folgt. Im Gegensatz dazu würde ohne krankhaft gesteigerte Interaktion der Neuronen keine Desynchronisation erfolgen.

Darüber hinaus kann durch die CR-Stimulation eine Neuorganisation der Konnektivität der gestörten neuronalen Netzwerke erzielt werden, so dass lang anhaltende therapeutische Effekte bewirkt werden können. Der erzielte synaptische Umbau ist von großer Bedeutung für die wirksame Behandlung neurologischer oder psychiatrischer Erkrankungen.

1. Messeinheit mit Sensoren: Die Sensoren messen Signale, welche es ermöglichen, adäquate Datenanalyse, insbesondere (a) ein phase locking, d.h. eine Phasensynchronisation, zwischen einem (bzgl. des timings) strikt periodischen Pulszug einerseits und der Phase der pathologischen oszillatorischen Aktivität andererseits und (b) eine Ab- bzw. Zunahme der Amplitude der pathologischen oszillatorischen Aktivität nachzuweisen, also z.B. Elektroenzephalographie (EEG)-Signale, Magnetenzephalographie (MEG)-Signale, lokale Feldpotentiale (LFP). Invasive Sensoren: implantierte Elektroden, z.B. epikortikale Elektroden, epidurale Elektroden, Tiefenelektroden Nicht-invasive Sensoren: (nicht implantierte) EEG-Elektroden (bevorzugte Ausstattungsvariante), MEG-Sensoren (SQUIDS). Weniger bevorzugt: indirekte Messung der neuronalen Aktivität durch Messung der damit einhergehenden Muskelaktivität mittels Elektromyographie (EMG).

2. Stimulationseinheit: Hierbei handelt es sich um eine Vorrichtung zur akustischen Stimulation, mit der Schallsignale ein- oder beidseitig sowie über beide Seiten unterschiedliche Schallsignale zu Stimulationszwecken (z.B. Töne, Klänge, Geräusche) über ein oder beide Ohren eines Patienten bzw. Probanden verabreicht werden.

3. Steuer- und Analyseeinheit: Diese Einheit verstärkt die gemessenen Signale. Die Steuer- und Analyseeinheit kann festverdrahtet vorliegen, beispielsweise als Rechner oder Controller oder dergleichen. Sie verfügt insbesondere über Datenanalyseverfahren zur Ermittlung der Ausprägung einer *Phasensynchronisation zwischen periodischem Reiz und zu untersuchendem neuronalen Rhythmus*, z.B.:

Zu den Zeiten $\tau_1$, $\tau_2$,..., $\tau_M$ wird ein Reiz appliziert. Es handelt sich hierbei um eine periodische Reizabfolge, d.h. die *Stimulationsperiode* (d.h. Periode der Reizabfolge) ist konstant: $T_{stim}$ = $\tau_{j+1}$ - $\tau_j$ für alle $j$ = 1, 2, ..., $M$ - 1, wobei $M$ die Anzahl der einzelnen Reize bezeichnet. Die zugehörige *Stimulationsfrequenz,* d.h. *Repetitionsrate* lautet $F_{stim}$ = 1/$T_{stim}$. Die Stimulationsfrequenz $F_{stim}$ wird gemäß dem Fachmann bekannten Vorwissen bzgl. der für die jeweilige Erkrankung charaktistischen pathologischen Frequenzbänder (also in Übereinstimmung mit den pathologischen Rhythmen, die mit CR desynchronisiert werden sollen) gewählt oder mittels Feedback durch Messung der zu desynchronisierenden pathologischen neuronalen Aktivität über Sensoren und Bestimmung des Frequenzpeaks im dem Fachmann bekannten pathologischen Frequenzband angepasst.

*Phase der periodischen Reizes:* Die Phase der periodischen Reizabfolge lautet $\varphi_1(t)$ = 2$\pi$ ($t$ - $\tau_1$)/$T_{stim}$ (siehe z.B. M.G. Rosenblum, A.S. Pikovsky, C. Schäfer, J. Kurths, P.A. Tass: Phase Synchronization: From Theory to Data Analysis. In: Moss F. (Ed.): Handbook of Biological Physics, Elsevier (2000)). Die so gewählte Phase verschwindet, wenn der ersten Reiz appliziert wird: $\varphi_1(t)$ = 0. Alternativ kann auch eine Phasenverschiebung "eingebaut" werden, die nichts an den Ergebnissen verändert, und auch keine Vorteile mit sich bringt. Eine Phasenverschiebung kann dadurch "eingebaut" werden, dass entweder eine andere Startzeit gewählt wird $\varphi_1(t)$ = 2$\pi$($t$ - $\xi$)/$T_{stim}$, wobei $\xi \neq \tau_1$ und $\xi$ = konst, oder indem eine Phasenverschiebung $\vartheta$ explizit dazu addiert wird $\varphi_1(t)$ = 2$\pi$($t$ - $\tau_1$)/$T_{stim}$ + $\vartheta$, wobei $\vartheta$ = konst.

*Phase der neuronalen Oszillation:* $\varphi_2$, die Phase des zu untersuchenden neuronalen Rhythmus wird mittels Hilbert-Transformation aus dem mittels Bandpassfilterung bzw. empirical mode decomposition bestimmten Signal, welches die pathologische oszillatorische Aktivität repräsentiert, ermittelt. Die empirical mode decomposition ermöglicht im Vergleich zur Bandpassfilterung eine parameterunabhängige Bestimmung physiologisch relevanter Moden in verschiedenen Frequenzbereichen (Huang N.E. et al. (1998) The empirical mode decomposition and the Hilbert spectrum for nonlinear and non-stationary time series analysis. Proc R Soc A: Math Phys Eng Sci 454:903-995). Die Kombination empirical mode decomposition mit nachfolgender Hilbert-Transformation wird als Hilbert-Huang-Transformation bezeichnet (AU Huang, NE et al. "A confidence limit for the empirical mode decomposition and Hilbert spectral analysis", Proceedings of the Royal Society of London Series A (2003), VOL. 459, 2317-2345). Neben der instantanen (zeitabhängigen) Phase $\varphi_2(t)$ der zu untersuchenden neuronalen Oszillation erhält man auf diese Weise zusätzlich deren instantane (zeitabhängige) Amplitude $A(t)$.

*Phasensynchronisation zwischen Reiz und neuronalem Rhythmus:*

Wir betrachten die n:m-Phasendifferenz zwischen dem Reiz und dem neuronalen Rhythmus $\psi_{n,m}(t)$ = $n\varphi_1(t)$ - $m\varphi_2(t)$, wobei $n$ und $m$ kleine ganze Zahlen sind, beispielsweise aus dem Bereich von 1 bis 5. Auf diese Weise kann die Phasensynchronisation zwischen dem Reiz und einem neuronalen Rhythmus in unterschiedlichen Frequenzbändern untersucht werden. D.h., zur Untersuchung des Effekts der Stimulation auf neuronale Rhythmen muss man sich nicht auf den Rhythmus, der sich im selben Frequenzbereich wie die Reizfrequenz ($n$ = $m$ = 1) befindet, beschränken. Die n:m-Phasendifferenz modulo $2\pi$ lautet $\phi_{n,m}(t)$ = [$n\varphi_1(t)$ - $m\varphi_2(t)$]$_{mod\ 2\pi}$

*n:m-Phasensynchronisation:*

Bestimmt u.a. durch die Abtastrate wird zu den Zeiten $t_1$, $t_2$, ..., $t_N$ die n:m-Phasendifferenz modulo $2\pi$ ermittelt.

Hiermit erhalten wir die zugehörige Verteilung von $\phi_{n,m}$, welche $\left\{\phi_{n,m}(t)\right\}_{j=a}^{b}$ lautet. Die Verteilung kann alle gemessenen Werte von $\phi_{n,m}$ beinhalten ($a$ = $t_1$, $b$ = $t_N$) oder nur eine Untergruppe ($a$ > $t_1$ und/oder $b$ < $t_N$) um z.B. Einschwingvorgänge von der Analyse auszuschließen. Hierzu werden z.B. die ersten ca. 10 Reize aus der Analyse herausgenommen.

Liegt keinerlei n:m-Phasensynchronisation vor, so ist die Verteilung der n:m-Phasendifferenz modulo $2\pi$ eine Gleichverteilung (bzw. kommt dieser hinreichend nahe). Im Gegensatz hierzu ist n:m-Phasensynchronisation durch das Auftreten von einem oder mehreren Häufungswerten von $\phi_{n,m}(t)$ charakterisiert (P. Tass, M.G. Rosenblum, J. Weule, J. Kurths, A. Pikovsky, J. Volkmann, A. Schnitzler, and H.-J. Freund: Detection of n:m Phase Locking from Noisy Data: Application to Magnetoencephalography. Phys. Rev. Lett. 81 (15), 3291-3294 (1998); M.G. Rosenblum, A.S. Pikovsky, C. Schäfer, J. Kurths, P.A. Tass: Phase Synchronization: From Theory to Data Analysis. In: Moss F. (Ed.): Handbook of Biological Physics, Elsevier (2000)).

Das Auftreten einer n:m-Phasensynchronisation kann mittels unterschiedlicher Variablen bestimmt werden. Im Folgenden werden Beispiele angeführt:

(i) Sollte die Verteilung von $\phi_{n,m}$ einen (einzigen) Häufungswert aufweisen, so kann der zirkuläre Mittelwert dieser

$$S_{n,m} = \left| \frac{1}{b-a+1} \sum_{l=a}^{b} exp[i\phi_{n,m}(t_l)] \right|,$$

Verteilung berechnet werden: wobei $0 \leq S_{n,m} \leq 1$, und eine Gleichverteilung zu $S_{n,m} = 0$ führt, während eine perfekte Phasensynchronisation durch $S_{n,m} = 1$ gekennzeichnet ist (M.G. Rosenblum, A.S. Pikovsky, C. Schäfer, J. Kurths, P.A. Tass: Phase Synchronization: From Theory to Data Analysis. In: Moss F. (Ed.): Handbook of Biological Physics, Elsevier (2000)). Der Nachteil dieses Synchronisationsindizes ist, dass er typischerweise keine sinnvollen Ergebnisse liefert, wenn die Verteilung $\{\phi_{n,m}(t)\}_{j=a}^{b}$ mehr als einen Häufungswert aufweist. Weist sie z.B. zwei gegenphasige Häufungswerte auf, so ergibt sich ein Wert von $S_{n,m}$ nahe Null.

(ii) Deswegen sollte zudem (oder ausschließlich) ein Synchronisationsindex berechnet werden, der unabhängig von der Anzahl der Häufungswerte verlässliche Ergebnisse liefert. Zu diesem Zweck wird mit dem Kuiper-Test, der zirkulären Variante des Kolmogorov-Smirnov-Tests (E. Batschelet, Circular Statistics in Biology (Academic Press, London, 1981); N. H. Kuiper: Tests concerning random points in a circle. Proc. K. Ned. Akad. Wet., Ser. A: Math. Sci. 63, 38 (1960); P.A. Tass: Transmission of stimulus-locked responses in two coupled phase oscillators. Phys.

Rev. E 69, 051909-1-24 (2004)), die Wahrscheinlichkeit bestimmt, mit der die Verteilung $\{\phi_{n,m}(t)\}_{j=a}^{b}$ eine Gleichverteilung ist. Man erhält dann einen P-Wert, welcher das kleinste Signifikanzniveau ist, mit dem die Nullhypothese (dass die beobachtete Verteilung $\{\phi_{n,m}(t)\}_{j=a}^{b}$ eine Gleichverteilung ist) zurückgewiesen kann. Ist

$\{\phi_{n,m}(t)\}_{j=a}^{b}$ eine Gleichverteilung, ergibt sich $P = 1$. Hat hingegen $\{\phi_{n,m}(t)\}_{j=a}^{b}$ einen einzelnen, deutlich ausgeprägten Häufungswert, so erhält man einen P-Wert nahe 0.

(iii) Eine weitere Möglichkeit ist ein auf der Shannon-Entropie der Verteilung $\{\phi_{n,m}(t)\}_{j=a}^{b}$ basierender n:m-

Synchronisationsindex $\rho_{n,m}$. Mit einer Abschätzung $p_k$ der Verteilung $\{\phi_{n,m}(t)\}_{j=a}^{b}$ ergibt sich

$$\rho_{n,m} = \frac{S_{max}-S}{S_{max}}, \quad \text{wobei} \quad S = -\sum_{k=1}^{L} p_k \ln p_k \cdot$$ Die maximale Entropie lautet $S_{max} = \ln L$, wobei $L$ die Anzahl der bins ist. $p_k$ ist die relative Häufigkeit, mit der $\phi_{n,m}$ im $k$-ten bin gefunden wird (P. Landa: Nonlinear Oscillations and Waves in Dyanmical Systems. Kluwer Academic Publishers, Dordrecht-Boston-London, 1996). Aufgrund der Normalisierung gilt $0 \leq \rho_{n,m} \leq 1$. Bei einer Gleichverteilung, also dem völligen Fehlen einer n:m-Phasensynchronisation ergibt sich $\rho_{n,m} = 0$, während eine perfekte n:m-Phasensynchronisation zu einer Dirac-artigen Verteilung führt (d.h., alle Werte von $\phi_{n,m}$ liegen im selben bin), so dass $\rho_{n,m} = 1$ . Im Vergleich zu dem unter (ii) angeführten, auf dem Kuiper-Test basierenden n:m-Synchronisationsindex hat der auf der Shannon-Entropie basierende n:m-Synchronisationsindex $\rho_{n,m}$ den Nachteil, dass sein Wert bei stärker ausgeprägten Häufungswerten der Verteilung von der genauen Lage des Häufungswertes abhängt, so dass sich bei einer zyklischen Verschiebung des Häufungswertes im Intervall $[0,2\pi]$ artefizielle Oszillationen ergeben (P.A. Tass: Transmission of stimulus-locked responses in two coupled phase oscillators. Phys. Rev. E 69, 051909-1-24 (2004)).

Neben den stimulationsbedingten Effekten auf die Phase $\varphi_2(t)$ des neuronalen Rhythmus und damit die n:m-Phasendifferenz $\phi_{n,m}$ werden auch stimulationsbedingte Effekte auf die Amplitude $A(t)$ des neuronalen Rhythmus untersucht. Es geht nicht nur darum, ob die Stimulation den neuronalen Rhythmus in einem n:m-Verhältnis in Takt bringt, sondern ob die Stimulation auch das Ausmaß der dem neuronalen Rhythmus zugrunde liegenden Synchronisation der Neuronen verändert. Ein Anstieg der Synchronisation innerhalb der stimulierten Neuronenpopulation führt zu einem Anwachsen von $A(t)$. Umgekehrt führt eine Abnahme der Synchronisation innerhalb der dem neuronalen Rhythmus zugrunde liegenden Neuronenpopulation zu einer Abnahme von $A(t)$. Für die Auswertung der Amplitudeneffekte sind somit Messungen in einem Vergleichsintervall (z.B. vor Verabreichung der Stimulation) durchzuführen. Alternativ können Amplitudeneffekte auch einfach durch eine Analyse der spektralen Leistungsdichte - eines vordefinierten, für die Erkrankung typischen und dem Fachmann bekannten Frequenzbandes oder einer bzw. mehrerer aus den Daten extrahierten empirical modes (s.o.) - bestimmt werden.

Bzgl. der Ausgestaltung der Erfindung ergeben sich u.a. 2 Varianten:

**(I) Kontinuierliche (bzw. kleinschrittige) Variation einer (oder mehrerer) Stimuluseigenschaft(en) während der Applikation eines streng periodischen Pulszugs:**

Wesentliches Kennzeichen dieses Verfahrens ist es, dass ein oder mehrere Reizparameter (z.B. die Tonhöhe) variiert werden, während ein periodischer Pulszug appliziert wird.

Beispiel: EEG-basierte Kalibration der CR-Therapietöne für die akustische CR-Stimulation zur Behandlung des Tinnitus sowie weiterer neurologischer und psychiatrischer Erkankungen (z.B. ADHS, Zwangserkrankungen, Depression)

Untervariante 1.1: Ermittlung der CR-Therapietöne ohne Zentralton

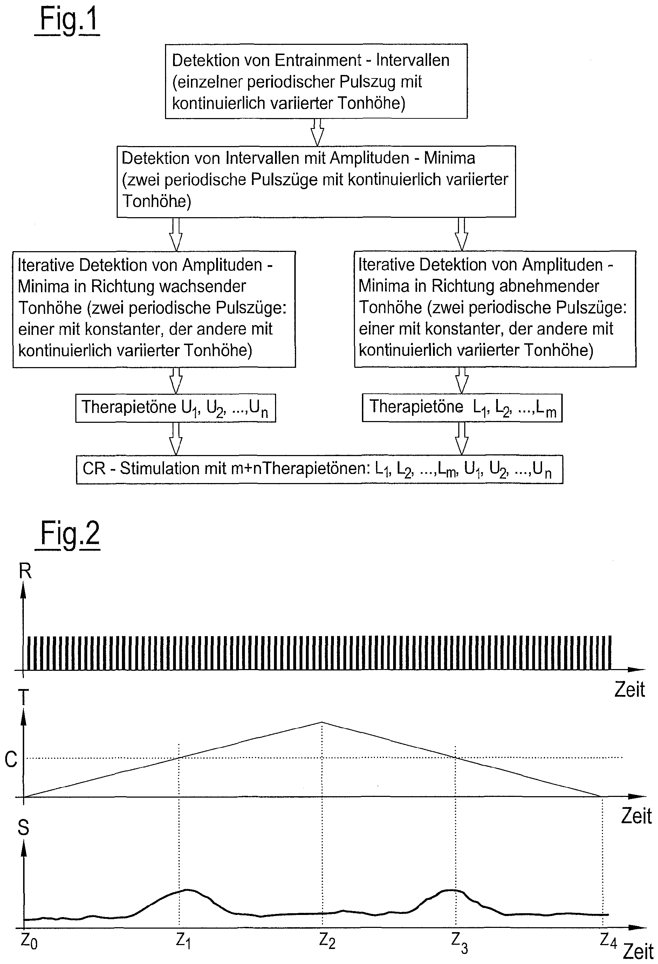

Hierbei handelt es sich um die bevorzugte Variante, welche von Beginn an eine symmetrische Anordnung der Therapietöne um das Zentrum des durch ein starkes Entrainment gekennzeichneten Bereichs (welcher dem Herd bzw. den Herden der krankhaften Synchronisation im zentralen Nervensystem entspricht) anstrebt. Der Ablauf eines beispielhaften Verfahrens ist in Fig. 1 schematisch dargestellt.

Fig. 1 zeigt ein Flussdiagramm des Ablaufs des erfindungsgemäßen Vorgehens bei der Variante ohne zentral angeordneten Therapieton (sog. Zentralton).

Fig. 2 zeigt die Detektion von Entrainment-Intervallen (einzelner periodischer Pulszug mit kontinuierlich variierter Tonhöhe). Die x-Achse ist in allen drei Abbildungen die Zeitachse. Die oberste Abbildung zeigt schematisch den Verlauf der akustischen Reize. Ein einzelner schwarzer Balken steht für einen reinen Ton der Tonhöhe T, welcher mit einem Hamming Window oder (weniger bevorzugt) mit einer Kosinus-Funktion oder sonstiger, bevorzugt glatter, den Ton typischerweise in seiner zeitlichen Ausdehnung beschränkender Einhüllenden gefaltet ist. Die mittlere Abbildung zeigt schematisch, wie die Tonhöhe T des periodisch applizierten Tons kontinuierlich variiert wird. Die untere Abbildung zeigt schematisch den Verlauf des Synchronisationsindex (in der Abbildung kurz als S bezeichnet), also z.B. des Phasensynchronisationsindex $S_{n,m}$ (s.o.).

I.1.a Detektion von Entrainment-Intervallen

Zuerst werden mittels eines (bzgl. der zeitlichen Struktur) konstanten Pulszugs mit kontinuierlich veränderter (z.B. in auf- und absteigender Richtungen variierter) Tonhöhe die Frequenzbereiche (d.h. Tonhöhenbereiche) detektiert, welche eine n:m-Phasensynchronisation zwischen Pulszug und Hirnoszillation (also ein n:m-Entrainment) aufweisen (Fig. 2). Bei einer n:m-Phasensynchronisation wird die pathologische neuronale Oszillation durch die Stimulation so beeinflusst, dass m Perioden der pathologischen Oszillation in n Perioden des periodischen Reizes zu liegen kommen (P. Tass, M.G. Rosenblum, J. Weule, J. Kurths, A. Pikovsky, J. Volkmann, A. Schnitzler, and H.-J. Freund: Detection of n:m Phase Locking from Noisy Data: Application to Magnetoencephalography. Phys. Rev. Lett. 81 (15), 3291-3294 (1998); M.G. Rosenblum, A.S. Pikovsky, C. Schäfer, J. Kurths, P.A. Tass: Phase Synchronization: From Theory to Data Analysis. In: Moss F. (Ed.): Handbook of Biological Physics, Elsevier (2000)). Die Phasendynamik einer pathologischen neuronalen Oszillation kann besonders effizient und mit geringer Intensität (Lautstärke) in den Tonhöhenbereichen beeinflusst werden, in denen eine ausgeprägte Phasensynchronisation nachgewiesen werden kann. Die in Fig. 2 gezeigten schematischen Ergebnisse können einerseits durch visuelle Inspektion ausgewertet werden. Vorzugsweise werden sie automatisch durch die erfindungsgemäße Vorrichtung ausgewertet. Z.B. können die zugehörigen Teile der Kurven des Synchronisationsindex (S in der untersten Abb. von Fig. 2), welche zum auf- und absteigenden Tonhöhenverlauf gehören, gemittelt werden:

$$\bar{S}(t) = [S(t) + S(z_4 - t)]/2 \, ,$$

wobei $t \in [z_0, z_2]$, wobei Anwachsen und Absinken der Tonhöhen mit gleicher Geschwindigkeit, also zeitlich symmetrisch, verlaufen (s. mittlere Abb. In Fig. 2), wobei $z_4 - z_2 = z_2 - z_0$. Die Zeitachse $t$ lässt sich im Falle eines linearen An- bzw. Abstiegs der Tonhöhe mit der Zeit einfach in eine Tonhöhenachse $g$ umrechnen. Wenn $g_0$ und $g_2$ die Tonhöhen sind, welche zu den Zeitpunkten $t_0$ und $t_2$ für den Pulszug verwendet werden, lautet die Umrechnungsformel $\bar{S}(g) = \bar{S}[z_0 + (z_2 - z_0)(g - g_0)/(g_2 - g_0)]$, wobei $g \in [g_0, g_2]$. Die akustische Coordinated Reset-Stimulation ist gegenüber Variationen der Tonhöhe der CR-Therapietöne hinreichend robust, so dass nicht das Maximum von $\overline{S}$ mit unangemessener (d.h. unrealistischer) Präzision zu detektieren ist. Vielmehr kann $\overline{S}(g)$ einfach tiefpass-gefiltert werden; die hierdurch erhaltene Kurve wird als $\overline{S}_T(g)$ bezeichnet. Die Parameter des Tiefpass-Filters sollten ermöglichen, dass sich das Maximum einfach bestimmen lässt. Die durch Variation der Parameter des Tiefpass-Filters entstehenden leichten Verschiebungen der Tonhöhe, bei der das Maximum von $\overline{S}_T(g)$ zu liegen kommt, beeinflussen den Therapieerfolg nicht. Die Tonhöhe, bei der das Maximum von $\overline{S}_T(g)$ detektiert wird, wird als *Zentralton C* bezeichnet: $\overline{S}_T(C) = \max_{g \in [g_0, g_2]}\{\overline{S}_T(g)\}$.

Sollte $\overline{S}_T(g)$ für $g \in [g_0, g_2]$ mehrere (genauer, *K* verschiedene) lokale Maxima (mit zugehörigen Tonhöhen, d.h. Zentraltönen $C_1, C_2, ..., C_K$) haben, gibt es unterschiedliche Ausgestaltungen, wie hierbei verfahren werden kann, z.B.:

(i) Berücksichtigt wird nur das (bei der Tonhöhe *C* gelegene) globale Maximum für $g \in [g_0, g_2]$, d.h. $\overline{S}_T(C) = \max_{j \in [1, ..., K]}\{\overline{S}_T(C_j)\}$.

(ii) Um Nebenpeaks eines Hauptmaximums bzw. "mehrfach gezackte" Hauptpeaks zu berücksichtigen, wird die *Spannweite* $\Delta S$ von $\overline{S}_T(g)$ *für* $g \in [g_0, g_2]$ berechnet: $\Delta S = \max_{g \in [g_0, g_2]}\{\overline{S}_T(g)\} - \min_{g \in [g_0, g_2]}\{\overline{S}_T(g)\}$. Für die weitere Auswertung sollen nur hinreichend ausgeprägte lokale Maxima berücksichtigt werden. Dies kann z.B. dadurch erreicht werden, dass nur lokale Maxima, welche z.B. die halbe Spannweite überschreiten, die also größer sind als $\sigma = \min_{g \in [g_0, g_2]}\{\overline{S}_T(g)\} + \gamma \Delta S$, mit $\gamma = 0.5$ berücksichtigt werden. Es können auch andere Werte von $\gamma$ gewählt werden. Es kann statt mit der Spannweite auch mit Größen, welche die Standardabweichung verwenden, gearbeitet werden. Um nicht nur den Wert eines lokalen Maximums $\overline{S}_T(C_j)$, also den Wert von $\overline{S}_T$ für eine einzelne Tonhöhe $C_j$ zu berücksichtigen, sondern eine repräsentativere Größe für ein lokales Maximum zu verwenden, können auch alle in einem zusammenhängenden Intervall $[g_a, g_b]$ die halbe Spannweite $\sigma$ überschreitenden Werte berücksichtigt werden, wenn also $\overline{S}_T(g) > \sigma$ für $g_a \leq g \leq g_b$ und $\overline{S}_T(g) \leq \sigma$ für $g_a - \varepsilon < g < g_a$ sowie für $g_b < g < g_b + \varepsilon$ mit hinreichend kleinem $\varepsilon$, z.B. wegen des über weite Bereiche der Tonhöhe relativen Charakters der Tonhöhenwahrnehmung: $\frac{g_b - g_a}{2}\xi$, wobei typischerweise $0 < \xi \leq 0.1$ gewählt wird, d.h. in einem Bereich von $100 \cdot \xi$ % um das Intervall $[g_a, g_b]$ darf $\overline{S}_T(g)$ die halbe Spannweite $\sigma$ nicht überschreiten. Statt eines zusammenhängenden Intervalls $[g_a, g_b]$ kann es bei manchen Anwendungen auch vorteilhaft sein, ein Intervall $[g_a, g_b]$ zu verwenden, welches Unterintervalle ç, enthält, welche $g_a < g_\alpha < g_b$ und $g_a < g_\beta < g_b$ erfüllen, die hinreichend klein sind: $g_\alpha - g_\beta < \delta$, wobei $\delta < \mu\varepsilon$ mit hinreichend großem $\mu$: $\mu > 1$. Überall in $[g_a, g_b]$ außer in den Unterintervallen $[g_\alpha, g_\beta]$ übersteigt $\overline{S}_T(g)$ die halbe Spannweite $\sigma$. Für alle innerhalb eines zusammenhängenden Intervalls $[g_a, g_b]$ (bzw. analog für alle in einem Intervall $[g_a, g_b]$ mit Unterintervallen $[g_a, g_b]$) liegenden Werte von $\overline{S}_T(g)$, welche die halbe Spannweite $\sigma$ überschreiten, kann nun z.B.

ein gewichtetes arithmetisches Mittel $\overline{C}_j = \dfrac{\sum_{k \in I_j} g_k \overline{S}_T(g_k)}{\sum_{k \in I_j} \overline{S}_T(g_k)}$ berechnet werden, wobei $I_j$ die Menge der Indizes des *j*-ten Intervalls $[g_a, g_b]$ ist. Zur Vermeidung von überladenen Formeln wurde der Index *j* in $[g_a, g_b]$ weggelassen. Es gilt also $I_j = \{a, ..., b\}$. Wir erhalten auf diese Weise ein oder mehrere (genauer: *L* verschiedene) lokale Maxima, die sich bei der/den Tonhöhe(n) $\overline{C}_1, ..., \overline{C}_L$ befinden.

Man kann nun auf verschiedene Weise fortfahren:

(a) Man passt die CR-Therapietöne nur für den obersten Zentralton $\overline{C}_L$ an, wobei $\overline{C}_L > \overline{C}j$ für alle $j = 1, ..., L - 1$. Sobald die Behandlung in diesem Tonhöhenbereich erfolgreich durchgeführt worden ist, kommt der nächst niedriger gelegen Zentralton, also $\overline{C}_{L-1}$.

(b) Man passt die CR-Therapietöne gemäß klinischen Kriterien an: Z.B. kann man mit dem Zentralton beginnen, welcher zu dem Tonanteil bzw. Geräuschanteil des Tinnitus gehört welcher am lautesten ist bzw., den Patienten am meisten stört. Nach erfolgreicher Behandlung folgt der Zentralton, der dem nächst lauten bzw. nächst störenden Tonanteil bzw. Geräuschanteil entspricht.

(c) Man selektiert den ersten zur Behandlung anstehenden Zentralton durch dynamische Marker, die im Rahmen der mit der erfindungsgemäßen Vorrichtung durchgeführten Untersuchung erzielt werden. Man kann z.B. folgende dynamische Marker verwenden:

(I) Man beginnt mit dem Zentralton, welcher zu dem größten Intervall $[g_a, g_b]$, in dem $\overline{S}_T(g)$ die halbe Spannweite $\sigma$ überschreitet, gehört. Von der Intervallbreite $g_b - g_a$ sollen die Bereiche der Unterintervalle $[g_\alpha, g_\beta]$ abgezogen werden, in denen $\overline{S}_T(g)$ die halbe Spannweite $\sigma$ nicht überschreitet. Nach erfolgreicher Therapie im Bereich dieses Zentraltons kommt der nächste Zentralton, der zu dem nächst breiten Tonhöhenintervall mit überschwelligem (d.h. die halbe Spannweite $\sigma$ übersteigendem) $\overline{S}_T(g)$ gehört.

(II) Man beginnt mit dem Zentralton, welcher in dem zugehörigen überschwelligen Intervall $[g_a,g_b]$ insgesamt (d.h. integral, also aufsummiert) das ausgeprägteste Entrainment aufweist. Hierzu wird für alle Zentraltöne $j = 1, ... , L$ die zugehörige integrale Stärke des Entrainments $\Lambda_j =$ berechnet: $\Lambda_j = \sum_{k\in I_j}\overline{S}_T(g_k)$. Nach erfolgreicher Therapie wird der Zentralton mit dem nächst stärksten Entrainment behandelt.

(III) Man beginnt mit dem Zentralton, welcher in dem zugehörigen überschwelligen Intervall $[g_a,g_b]$ das ausgeprägteste relative Entrainment (in Relation zur Ausdehnung des überschwelligen Bereichs) aufweist. Hierzu wird für alle Zentraltöne $j = 1, ..., L$ die zugehörige relative Stärke des Entrainments $\Upsilon_j =$ berechnet:

$$\Upsilon_j = \overline{C}_j = \frac{\sum_{k\in I_j}\bar{S}_T(g_k)}{\overline{B}_j},$$

wobei die $B_j$ die Ausdehnung des zum $j$-ten Zentraltons gehörigen überschwelligen Bereichs ist. Im einfachen Fall, wenn $[g_a,g_b]$ keine unterschwelligen Intervalle $[g_\alpha,g_\beta]$ enthält, gilt $B_j = g_b - g_a$. Nach erfolgreicher Therapie wird der Zentralton mit dem nächst stärksten Entrainment behandelt.

(IV) Man bestimmt für alle Zentraltöne die zugehörigen CR-Therapietöne und behandelt mit diesen alle Zentraltöne zeitlich voneinander getrennt und sukzessive, wobei die Behandlungsdauer für den einzelnen Zentralton an klinische Marker (d.h. an die Lautheit des entsprechenden Tonanteils bzw. Geräuschanteils am Tinnitus bzw. an die Belästigung durch den entsprechenden Tonanteil bzw. Geräuschanteil) oder an die oben definierten dynamischen Marker $(B_j, \Lambda_j$ oder $\Upsilon_j)$ angepasst werden kann, z.B. durch eine lineare Korrelation: je lauter der entsprechende zentralton, desto länger wird er behandelt. D.h., Zentralton 1 wird mit den zugehörigen CR-Therapietönen behandelt während einer gewissen Zeitdauer behandelt. Danach wird Zentralton 2 ggfs. während einer anderen Zeitdauer behandelt. Die Reihenfolge, in der die verschiedenen Zentraltöne behandelt werden, kann stochastisch oder deterministisch (z.B. chaotisch) oder gemischt stochastisch/chaotisch variiert werden. Die Reihenfolge kann aber auch stets oder in einem gewissen, z.B. durch Randbedingungen in einem Zufallsprozess, realisierten Rahmen von den klinischen Markern (Lautheit und/oder Belästigung) und/oder den dynamischen Markern $(B_j, \Lambda_j$ oder $\Upsilon_j)$ beeinflusst werden bzw. vorgegeben sein.

(V) Für alle Zentraltöne $\overline{C}_1, ..., \overline{C}_L$ werden die zugehörigen CR-Therapietöne bestimmt. In der weiteren funktionalen Austestung werden nur die CR-Therapietöne für die CR-Therapie verwandt, welche tatsächlich die funktionalen Kriterien (s.u.) erfüllen. Mit anderen Worten, wenn die unterschiedlichen Hirngebiete, die zu den verschiedenen überschwelligen Intervalle (und damit zu den unterschiedlichen Zentraltönen $\overline{C}_1, ..., \overline{C}_L$) gehören, anatomisch hinreichend stark synaptisch miteinander verbunden sind, dass sich entsprechende Befunde in der funktionalen Austestung (s.u.) ergeben, werden alle zugehörigen CR-Therapietöne für die Behandlung verwandt. Sollten sich zwei oder mehrere Hirngebiete, die zu unterschiedlichen Zentraltönen gehören, als nicht hinreichend miteinander synaptisch verbunden erweisen (s.u.), wird nur einer der beiden Zentraltöne behandelt. Die Priorisierung bei der Behandlung der jeweiligen Zentraltöne erfolgt wie oben beschrieben.

Alternativ zu der oben beschriebenen Methode für die Maximumsbestimmung kann das Maximum bzw. können die Maxima von $\overline{S}$ auch mittels anderer dem Fachmann bekannter Testverfahren (z.B. Verfahren, die in der Literatur und in den Anwendungen unter dem Begriff "Change-Point Analyzer" firmieren, siehe z.B. Taylor, Wayne (2000a), Change-Point Analyzer 2.0 shareware program, Taylor Enterprises, Libertyville, Illinois. Web: http://www.variation.com/cpa) bestimmt werden.

Die Stärke der n:m-Phasensynchronisation zwischen Pulszug und Hirnoszillation hängt von der Lautstärke der zur Stimulation verwandten Töne ab. Hierzu gibt es nun mehrere Varianten, wie die erfindungsgemäße Vorrichtung verfahren kann:

1. Im Rahmen einer für die praktische Durchführung vorteilhaften, da schnell und einfach durchführbaren Variante wird eine *Lautstärke voreingestellt* und während der gesamten Untersuchung (und insbesondere während der in Figs. 2ff skizzierten Schritte) *konstant* gehalten. Dies ist vor allem bei Tinnituspatienten kein bevorzugtes Vorgehen. Ein solches, schnelles Vorgehen kann bei Patienten, die nicht an einem (typischerweise mit Hörschäden einhergehenden) Tinnitus, sondern z.B. an einer Depression leiden, die mit Tönen im Sprachbereich (und nicht im Hochtonbereich, in dem sich häufiger Hörschäden finden) stimuliert werden und bei denen in einer vorgeschalteten Schwellen-Audiometrie ein pathologischer Befund (also ein Hörschaden) ausgeschlossen werden konnte, in Betracht gezogen werden. Die voreingestellte Lautstärke sollte entweder schwellennahe oder bis zu 20 dB oberhalb der Hörschwelle, ggfs. (falls das Entrainment schlecht durchführbar sein sollte) noch größer gewählt werden.

2. Die Lautstärke der zur Stimulation verwandten Töne wird durch eine Interpolation und Extrapolation aus einem klassischen Schwellenaudiogramm ermittelt. Diese Variante ist auch schnell und dadurch sehr praktikabel durchführbar. Bei einem klassischen Schwellenaudiogramm wird die Hörschwelle an vergleichsweise wenigen Stützstelle ermittelt. Bzgl. der Interpolation birgt dieses Vorgehen Fehlermöglichkeiten, da es prinzipiell, aber nicht beliebig häufig schmale Hörsenken gibt. Die Ausdehnung dieser Hörschäden kann im Frequenzraum so eng umschrieben sein, dass diese schmalen Hörsenken infolge die wenigen Stützstellen des Schwellenaudiogramms nicht erfasst werden können. Die Lautstärke der Stimuli mit Frequenzen im Bereich solch einer Hörsenke müsste angehoben werden, um den Hörschaden zu kompensieren. Auf diese Weise kann es passieren, dass gerade im Bereich einer Hörsenke, die z.B. bei Tinnituspatienten auch gehäuft der Frequenzbereich des Tinnitus ist, eine (im Vergleich zur Hörschwelle) deutlich zu schwache Stimulation verwandt wird, so dass kein Entrainment (Fig. 2) beobachtet werden kann. Bzgl. der Extrapolation birgt diese Variante die Schwierigkeit, dass oberhalb des höchsten Stützpunkts des Schwellenaudiogramms eine Hochtonschwerhörigkeit vorhanden ist. Auch in diesem Fall wären die Stimulationstöne dann in diesem (extrapolierten) Bereich nicht laut genug, und es ergäbe sich ein irreführendes Ergebnis.

3. Um die Hörschwelle detailliert/engmaschig genug abzurastern (auszumessen), kann insbesondere bei Patienten, die eine Hörminderung bzw. einen Hörschaden (z.B. gemäß Standard-Schwellenaudiometrie) aufweisen oder bei Patienten, die mit den beiden vorgenannten Varianten keine hinreichend guten Ergebnisse erzielt werden konnten, oder aus sonstigem medizinischen Grund der Verdacht einer schmalbandigen Hörsenke vorliegt, folgende Variante verwandt werden:

Vor der in Fig. 2 illustrierten Messung wird zuerst eine Bekesy-Audiometrie (Békésy G. v.: A new audiometer. Acta oto-laryngol. (Stockh.) 35 (1947) 411; Lehnhardt E., Laszig R.: Praxis der Audiometrie. Thieme, Stuttgart, 9. Auflage, 2009) durchgeführt, um kontinuierlich die Hörschwelle über die Frequenzachse abzurastern. Hierzu wird bei der Hörmessung die Frequenz (Tonhöhe) stufenlos kontinuierlich von z.B. 100 Hz bis 10000 Hz variiert. Bei Tinnituspatienten mit hochfrequentem Tinnitus (z.B. > 8000 Hz) bzw. einer Hochtonschwerhörigkeit wird die Frequenz noch weiter nach oben (z.B. bis 130000 Hz oder 16000 Hz) innerhalb von z.B. 400 Sekunden oder 200 Sekunden variiert. Die Intensität wird bis zu 120 dB in kleinen, 0,25 dB-Stufen und mit einer Geschwindigkeit von z.B. 2,5 dB/sec oder 5 dB/sec geregelt. Der Ton wird entweder als Dauerton oder Impulston (von 200ms oder 150 ms Dauer) verabreicht. Die bevorzugte Variante ist die Impulstonmethode, da sie die Hörschwelle für Impulstöne ermittelt, und Impulstöne zur weiteren Untersuchung des Entrainments verwandt werden. Als Puls-Pausenverhältnis (also als Verhältnis zwischen der Dauer des einzelnen Impulstons und der nachfolgenden Pause) können Verhältnisse wie 1:1 oder 1:3 gewählt werden. Bei der Impuls-Variante kann auch die Stimulationsfrequenz (Repetitionsrate, mit der der Impulston verabreicht wird) an den dominanten spektralen Frequenzpeak der über die Sensoren (z.B. EEG-Elektroden) gemessene pathologische Oszillation in einem kleinen ganzzahligen n:m-Verhältnis angepasst werden (wobei n und m kleine ganze Zahlen sind).

Der Patient regelt über einen Handschalter (dB-Regler) den Lautstärke-Abschwächer. Solange der Handschalter gedrückt ist, geht die Lautstärke des Tones zurück. Wird der Handschalter nicht gedrückt, nimmt die Lautstärke wieder zu. Die Intensität (Lautstärke) wird dabei (wie oben erwähnt) mit einer Geschwindigkeit von z.B. 2,5 dB/sec oder 5 dB/sec in 0,25 dB-Stufen geregelt. Es können auch andere Stufenwerte, z.B. 2 dB, gewählt werden. Weniger bevorzugt kann auch eine völlig stufenlose Regelung der Intensität gewählt werden. Man erhält hierdurch eine zackenförmig verlaufende Kurve, da sich die vom Patienten geregelte Hörschwelle zwischen einem gerade schon hörbaren und einem nicht mehr hörbaren Intenstitätspegel hin- und herbewegt.

Wenn die **Békésy-Audiometrie separat,** vor der in Fig. 2 skizzierten Entrainment-Untersuchung **durchgeführt** wird, wird eine in Abhängigkeit von der Frequenz (d.h. von der Tonhöhe T) kontinuierliche Hörschwelle dadurch ermittelt, dass die lokalen Extrema, welche zu den gerade schon hörbaren Intensitätspegeln gehören, interpoliert (und nach hohen Werten ggfs. extrapoliert) werden. Die Intensitätspegel dieser Hörschwelle werden als *kontinuierliche Hörschwelle* bezeichnet und als Intensitätswerte für den periodischen Pulszug aus Fig. 2 gewählt. Alternativ kann auch unabhängig von der jeweiligen Frequenz (d.h. Tonhöhe T) ein konstanter Intensitätspegel von z.B. 2 dB oder 3 dB oder 5 dB oder größeren Werten wie 10 dB oder sogar 20 dB oder mehr zu der Hörschwelle hinzuaddiert werden. Diese Kurve wird als *kontinuierliche Intensitätsschwelle* bezeichnet. Die Werte der kontinuierlichen Intensitätsschwelle werden als Intensitätswerte für den periodischen Pulszug aus Fig. 2 verwendet. Derart erhöhte Intensitätswerte können vorteilhaft sein, wenn mit den der Hörschwelle entsprechenden Intensitätswerten kein hinreichend starkes Entrainment hervorgerufen werden kann.

Statt der kontinuierlichen Intensitätsschwelle kann auch eine Isophone bestimmt werden. Da dies aber typischerweise nur an einer begrenzten Anzahl von Frequenzen (Tonhöhen) erfolgt, so dass eine Interpolation und (an der oberen Grenze) eine Extrapolation nötig ist, ist deren Wert, sofern sie nicht auf zeitaufwändige und detaillierte Weise erstellt werden sollte, typischerweise begrenzt. Für die Zwecke der erfindungsgemäßen Vorrichtung reicht eine kontinuierliche Intensitätsschwelle typischerweise vollkommen aus.

Wenn die **Bekesy-Audiometrie gleichzeitig** mit der in Fig. 2 skizzierten Entrainment-Untersuchung **durch-**

**geführt** wird, kann (je nach Stimulationsfrequenz $F_{stim}$) eine längere Untersuchungsdauer als bei der alleinigen Bekesy-Audiometrie gewählt. Dies ist nötig, um für das jeweilige TonhöhenIntervall (in dem ein Entrainment detektiert werden soll) eine ausreichende Anzahl von Stimulationsperioden $T_{stim}$ zur Berechnung des Phasensynchronisationsindex $S_{n,m}$ zu erhalten. Die angestrebte Genauigkeit der *Frequenzauflösung* (d.h. *Tonhöhen-Auflösung*) wird vor allem durch folgenden Parameter bestimmt:

*Anzahl der Stimulationsperioden $N_{stim}$* pro *Fensterbreite* $\Delta F$ (Differenz der oberen und unteren Tonhöhe in Hz des Fensters). Dieser Parameter bestimmt die *Untersuchungsdauer $T_{ges}$.*

Die Stimulationsfrequenz (Repetitionsrate) $F_{stim}$ und damit die Stimulationsperiode $T_{stim}$ richten sich nach der *dominanten Frequenz $\hat{F}$* (d.h. dem Frequenzpeak im Spektrum) der pathologischen neuronalen kollektiven (synchronisierten) Oszillation, welche desynchronisiert werden soll. Im günstigsten Fall wird die Stimulationsfrequenz $F_{stim}$ so gewählt, dass sie der dominanten Frequenz möglichst ähnlich ist, d.h. $F_{stim} \approx \hat{F}$, wobei $F_{stim}$ von $\hat{F}$ nicht wesentlich mehr als 5 % oder 10 % oder 20 % abweichen sollte. Das Verfahren ist robust, da in für die praktische Anwendung hinreichendem Ausmaß ein Abweichen der beiden Frequenzen durch einen Zuwachs der Lautstärke der Reize kompensiert werden kann. Es sollte aber mit möglichst geringer Lautstärke gearbeitet werden, da nur dann die räumliche Stimulationseinwirkung im Gehirn räumlich sehr selektiv ist, und manche Patienten zu laute Reize als unangenehm und ermüdend empfinden.

In einer weniger bevorzugten Ausführung der erfindungsgemäßen Vorrichtung wird $F_{stim}$ nicht an $\hat{F}$ angepasst, sondern für die dem Fachmann bekannten Frequenzbänder eine Frequenz a priori gewählt. Sollte die Abweichung der Stimulationsfrequenz $F_{stim}$ von der dominanten Frequenz $\hat{F}$ so groß sein, dass die Ergebnisse des Entrainments zu schwach ausgeprägt sein sollten, kann dies durch eine Erhöhung der Lautstärke kompensiert werden. Hierzu wird z.B. zur Hörschwelle (die wie unten beschrieben bestimmt wird) ein fixer Betrag von z.B. 5 dB hinzuaddiert.

Die Tonhöhe wird stufenlos kontinuierlich und linear (d.h. mit konstanter Geschwindigkeit) von $F_{unten}$ bis $F_{oben}$, z.B. von $F_{unten}$= 100 Hz bis $F_{oben}$ = 13000 Hz, variiert. Die Tonhöhe kann (in einer anderen Ausgestaltung) auch nicht-linear, z.B. logarithmisch anwachsen. Im Fall des linearen Anwachsens der Tonhöhe wird die Untersuchungsdauer $T_{ges}$ wie folgt berechnet:

$$T_{ges} = \frac{F_{oben} - F_{unten}}{\Delta F} N_{stim} T_{stim},$$

wobei $N_{stim} T_{stim}$ die Zeit zum Durchlaufen der Fensterbreite $\Delta F$ mit $N_{stim}$ Stimulationsperioden der Dauer $T_{stim}$ ist.

Beispiel: Mit den Parametern $N_{stim}$= 100, $\Delta F$ = 500 *Hz*, $F_{unten}$= 100 Hz, $F_{oben}$ = 13000 Hz ergibt sich im Falle einer *dominanten Frequenz $\hat{F}$* = 1.5 Hz eine Stimulationsfrequenz $F_{stim}$ = 1.5 Hz und somit eine Stimulationsperiode $T_{stim}$ = 0,666 s. Somit ergibt sich als Untersuchungsdauer $T_{ges}$=

$$\frac{F_{oben} - F_{unten}}{\Delta F} N_{stim} T_{stim} = \frac{F_{oben} - F_{unten}}{F_{stim} \Delta F} N_{stim} = 28,7 \text{ min.}$$

Wenn man die Fensterbreite $\Delta F$ = 500 *Hz* hingegen nur mit $N_{stim}$= 50 Stimulationsperioden abscannen möchte, wird man eine weniger gute Trennschärfe zwischen den Frequenzbereichen mit bzw. ohne Entrainment erlangen, dafür aber die Untersuchungsdauer halbieren: $T_{ges}$= 14,3 min.

Die dominante Frequenz $\hat{F}$ = 1.5 Hz liegt im unteren Bereich des sogenannten Delta-Frequenzbands (1-4 Hz). Falls die dominante Frequenz größer ist, ergibt sich eine deutlich kürzere Untersuchungsdauer. Z.B. erhält man für $\hat{F}$ = 3 Hz = $F_{stim}$ (und sonst gleichen Parametern) die Untersuchungsdauer $T_{ges=}$ 14,3 min für $N_{stim}$= 100 und $T_{ges}$= 7,2 min für $N_{stim}$= 50. Liegt die dominante Frequenz im Theta-Frequenzband (5-8 Hz) und beträgt z.B. $\hat{F}$ = 6 Hz, erhalten wir mit $F_{stim} = \hat{F}$ die zugehörige Untersuchungszeit $T_{ges}$= 7,2 min für $N_{stim}$= 100 und $T_{ges}$= 3,6 min für $N_{stim}$= *50*.

Die Stimulationsfrequenz bestimmt ganz entscheidend die Untersuchungsdauer. Um die Untersuchungszeit $T_{ges}$ möglichst klein zu halten, kann zum einen die Anzahl der Stimulationsperioden pro Fensterbreite $N_{stim}/\Delta F$ gering gewählt werden. Zum anderen bzw. zusätzlich kann die Stimulationsfrequenz $F_{stim}$ auch größer als die dominante Frequenz $\hat{F}$ gewählt werden. Dies führt insbesondere dann zu einer Verkürzung der Untersuchungszeit $T_{ges}$, wenn die dominante Frequenz $\hat{F}$ im Delta-Frequenzband (1-4 Hz) und nicht im Theta-Frequenzband (5-8 Hz) liegt. Falls die dominante Frequenz $\hat{F}$ gemessen wird, kann die Stimulationsfrequenz $F_{stim}$ so gewählt werden, dass sie in einem kleinen ganz-

$$\frac{F_{stim}}{\hat{F}} = \frac{n}{m} > 1,$$

zahligen Verhältnis zu $\hat{F}$ steht: wobei $n$ und $m$ kleine ganze Zahlen sind. Mit abnehmender Untersuchungsdauer kann allerdings die Güte der Abtastung der Hörschwelle abnehmen. Dies ist allerdings kein wirklich gravierender Punkt, da die Ergebnisse hinreichend stabil sind gegenüber einer Variation der Lautstärke der Stimulationstöne.

Zusammengefasst ergeben sich folgende Varianten für das von der erfindungsgemäßen Vorrichtung realisierte beispielhafte Verfahren zur Bestimmung der n:m-Phasensynchronisation zwischen Pulszug und Hirnoszillation:

- die Lautstärke der Reize ist voreingestellt (wenig bevorzugt)
- die Lautstärke wird an ein konventionelles Audiogramm (das an wenigen Stützstellen bestimmt wird) angepasst; hierzu kann der kontinuierliche Verlauf der Hörschwelle durch Interpolation und Extrapolation approximiert werden.
- Ein Békéshy-Audiogramm liefert die quasi kontinuierliche Hörschwelle. Wird das Bekeshy-Audiogramm nicht parallel zur Entrainment-Untersuchung durchgeführt, kann eine kontinuierliche Hörschwelle durch Interpolation (und ggfs. an der oberen Grenze auch durch Extrapolation) der lokalen Extrema des (zackenförmigen) Bekeshy-Audiogramms erstellt werden, welche als gerade hörbar empfunden werden. Das Bekeshy-Audiogramm kann aber auch eleganterweise parallel zur Entrainment-Untersuchung durchgeführt werden. Auf diese Weise kann die Untersuchungszeit relevant verkürzt werden.
- Sollten die Intensitäts- (Lautstärken-) Werte der Hörschwelle (bestimmt durch ein klassisches Audiogramm oder durch eine Bekeshy-Audiometrie) kein hinreichend starkes entrainment (also keine hinreiched starke n:m-Phasensynchronisation zwischen Pulszug und Hirnoszillation ermöglichen, kann eine stärker Reizintensität entweder (bevorzugt) durch Verwendung einer kontinuierlichen Intensitätsschwelle oder einer Isophone (s.o.) gewählt werden. In Abhängigkeit vom Ergebnis kann die Abweichung der kontinuierlichen Intensitätsschwelle bzw. der Isophone von der Hörschwelle erhöht werden, bis schließlich ein hinreichend starkes entrainment erreicht wird.

Dieses Vorgehen stiftet einen Zentralton C, also eine zentrale Frequenz (im Sinne einer Tonhöhe) C, welche als anatomisches Zentrum eines synchronen Fokus im zentralen auditorischen System (z.B. im auditorischen Cortex) angesehen werden kann.

I.1.b Detektion von Intervallen mit Amplituden-Minima

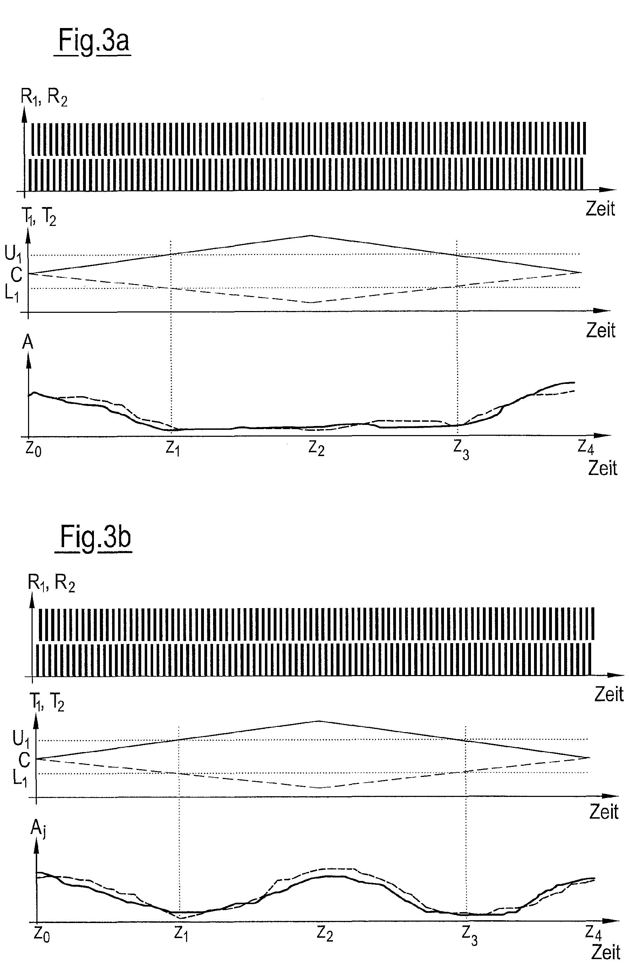

Ziel dieses Schrittes ist es, die optimale Distanz zwischen benachbarten Therapietönen zu bestimmen. Hierzu werden zwei (zeitlich) konstante periodische phasenverschobene, insbesondere gegenphasige Pulszüge appliziert, wobei die Tonhöhe beider Pulszüge kontinuierlich und gegenläufig variiert wird (Fig. 3). Liegt die Tonhöhe beider Pulszüge nahe beieinander, wird durch beide Pulszüge im Wesentlichen (d.h. mit einem großen räumlichen Überlapp) die selbe Region in der Hörrinde (bzw. dieselben Regionen in verschiedenen nachgeschalteten Gebieten der zentralen Hörbahn) im Gehirn stimuliert. Dieser Überlapp rührt daher, dass - wie durch die sogenannten Tuning-Kurven beschrieben - benachbarte Neuronen zwar durch Schall unterschiedlicher Frequenz optimal angeregt werden, aber durch Schall benachbarter Frequenzen auch noch, wenn auch typischerweise schwächer angeregt werden. In diesem Fall wird im Wesentlichen eine periodische Stimulation mit doppelter Stimulationsfrequenz $F_{stim}$ durchgeführt. Liegt die Tonhöhe beider Pulszüge zu weit auseinander, so dass die beiden angeregten Hirngebiete infolge der mit der Distanz abnehmenden Stärke der anatomischen Vernetzung nicht mehr hinreichend stark interagieren (anatomisch gekoppelt sind), so werden zwei unterschiedliche Orte im Gehirn phasenverschoben (gegenphasig) angeregt. Wenn das von beiden angeregten Neuronenpopulationen erzeugte elektrische Feld exakt dieselbe Amplitude haben sollte, können sich die Felder beider Neuronenpopulationen - je nach anatomischer Lage der dipolartig angeordneten Neuronen - zumindest teilweise aufheben. In diesem Fall verschwindet in erster Näherung des gesamte elektrische Feld im Bandpass-Frequenzbereich der einfachen und doppelten Stimulationsfrequenz $F_{stim}$. Sollte die Anregung bei beiden Neuronenpopulationen (z.B. infolge unterschiedlicher Größe der Neuronenpopulationen und/oder unterschiedlicher anatomischer Ausrichtung der jeweils beteiligten Neuronen etc.) nicht zu einem gleich starken Feld führen, ergibt sich ein in Summe von Null verschiedenes Feld, was im Bandpassbereich der einfachen und doppelten Stimulationsfrequenz $F_{stim}$ zu entsprechend von Null verschiedenen Werten führt (Fig. 3b). Die geringsten Werte der Amplitude - und somit der Bandpassbereiche welche der einfachen und doppelten Stimulationsfrequenz $F_{stim}$ entsprechen - des von beiden Neuronenpopulationen generierten elektrischen Feldes ergibt sich, wenn die beiden Neuronenpopulationen anatomisch interagieren (also nicht zu weit von einander entfernt lokalisiert sind) und durch die gegenphasige Stimulation, also CR-Stimulation, desynchronisiert werden (Figs. 3a, 3b).

In Analogie zur obigen Fragestellung (Fig. 2), bei der Maxima detektiert wurden, erfolgt hier nun (mit denselben Methoden) eine Minimums-Detektion (Fig. 3b) oder eine Detektion der Ränder eines ausgedehnten Bereichs geringer Werte (Fig. 3a). Dies kann mit Datenanalyse-Verfahren, die dem Fachmann bekannt sind, durchgeführt werden. Es kann z.B. die für die Maximumsdetektion (Fig. 2) verwandte Mittelungstechnik verwandt werden und anstatt eines Maximums (wie in Fig. 2) nun ein Wendepunkt detektiert werden.

Fig. 3a zeigt die Applikation zweier konstanter periodischer gegenphasiger Pulszüge, wobei die Tonhöhe beider Pulszüge kontinuierlich und gegenläufig variiert wird. Die x-Achse ist in allen drei Abbildungen die Zeitachse. Die oberste Abbildung zeigt schematisch den Verlauf der beiden akustischen Reizabfolgen. Ein einzelner schwarzer Balken steht für einen reinen Ton der Tonhöhe T, welcher mit einem Hamming Window oder (weniger bevorzugt) mit einer Kosinus-Funktion oder sonstiger, bevorzugt glatter, den Ton typischerweise in seiner zeitlichen Ausdehnung beschränkender Einhüllenden gefaltet ist. Die mittlere Abbildung zeigt schematisch, wie die Tonhöhen $T_1$ und $T_2$ der beiden periodischen Reizabfolgen kontinuierlich variiert werden. Die untere Abbildung zeigt schematisch den Verlauf der Amplitude (z.B. Hilbert-Amplitude oder Integral der Power im untersuchten Frequenz-Bandpass) welche zur Stimulationsfrequenz $F_{stim}$ ($A_1$) bzw. der doppelten Stimulationsfrequenz ($A_2$) gehört.

Fig. 3b zeigt die Applikation zweier konstanter periodischer gegenphasiger Pulszüge, wobei die Tonhöhe beider Pulszüge kontinuierlich und gegenläufig variiert wird. Format wie in Fig. 3a.

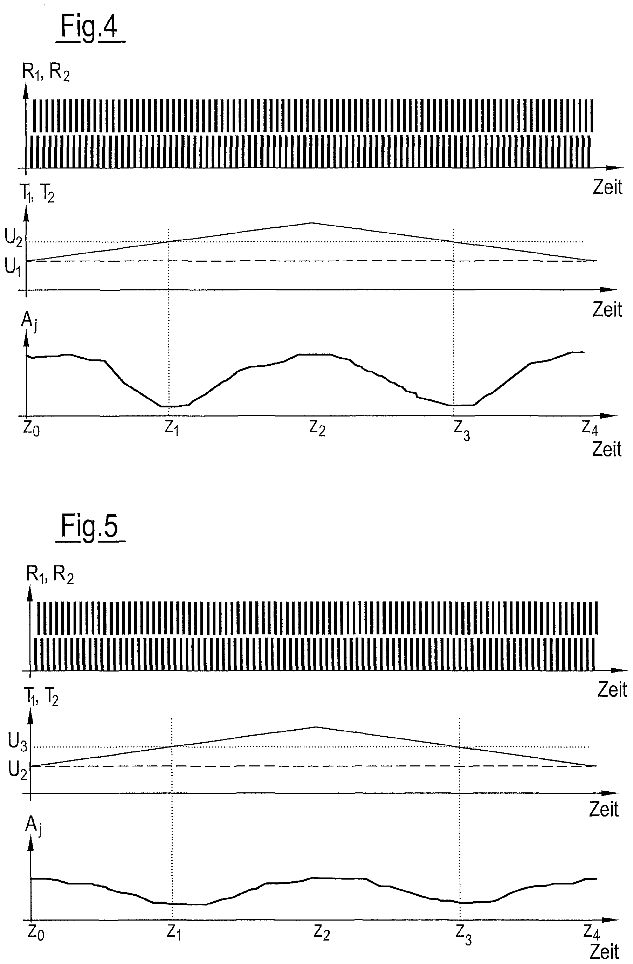

Durch dieses Vorgehen erhält man einen oberhalb des Zentraltons C gelegenen Therapieton $U_1$ sowie einen unterhalb des Zentraltons C gelegenen Therapieton $L_1$. Nun werden iterativ weitere mögliche Therapietöne oberhalb des Zentraltons C ($U_2$, $U_3$, ...) bzw. unterhalb des Zentraltons C ($L_2$, $L_3$, ...) ermittelt, indem zwei gegenphasige Pulsfolgen verabreicht werden, wobei die Tonhöhe der einen Pulsfolge konstant beim jeweiligen Ausgangs-Therapieton (z.B. $U_1$ in Fig. 4) liegt und die Tonhöhe der anderen Pulsfolge zur Detektion des nächst höheren Therapietons zuerst anwächst und dann wieder sinkt (Fig. 4). In Analogie zu Fig. 3a bzw. 3b detektieren wir (wie oben beschrieben) die Minima (Fig. 4) der Amplitude des Bandpasses, welcher der einfachen oder doppelten Stimulationsfrequenz $F_{stim}$ entspricht. Dies wird so lange iterativ durchgeführt, wie relevante Minima (Fig. 5) bzw. Wendepunkte detektierbar sind. Hierbei können verschiedene Kriterien für die Relevanz verwandt werden. Z.B. werden nur Minima akzeptiert, welche hinreichend stark von Mittelwert und Standardabweichung differieren. Dem Fachmann stehen hier mehrere Verfahren der Datenanalyse sowie statistische testverfahren zur Verfügung.

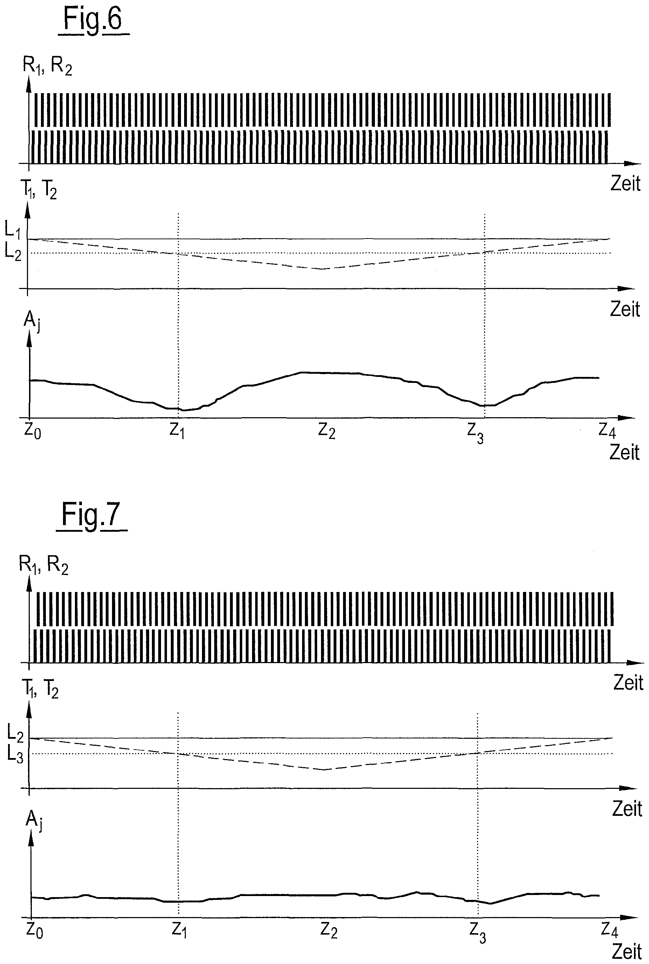

In Analogie der jeweils nächst höher gelegenen Therapietöne $U_2$, $U_3$,... werden auch die jeweils nächst niedriger gelegenen Therapietöne L2 (Fig. 6), L3 (Fig. 7),... detektiert.

Fig. 4 zeigt die Applikation zweier konstanter periodischer gegenphasiger Pulszüge, wobei die Tonhöhe ($U_2$) nur eines der beiden Pulszüge kontinuierlich in auf- und danach absteigender Weise variiert wird. Die Tonhöhe ($U_1$) des anderen Pulszugs bleibt konstant. Format wie in Fig. 3a.

Fig. 5 zeigt die Applikation zweier konstanter periodischer gegenphasiger Pulszüge, wobei die Tonhöhe ($U_3$) nur eines der beiden Pulszüge kontinuierlich in auf- und danach absteigender Weise variiert wird. Die Tonhöhe ($U_2$) des anderen Pulszugs bleibt konstant. Format wie in Fig. 3a.

Fig. 6 zeigt die Applikation zweier konstanter periodischer gegenphasiger Pulszüge, wobei die Tonhöhe ($L_2$) nur eines der beiden Pulszüge kontinuierlich in ab- und danach aufsteigender Weise variiert wird. Die Tonhöhe ($L_1$) des anderen Pulszugs bleibt konstant. Format wie in Fig. 3a.

Fig. 7 zeigt die Applikation zweier konstanter periodischer gegenphasiger Pulszüge, wobei die Tonhöhe ($L_3$) nur eines der beiden Pulszüge kontinuierlich in ab- und danach aufsteigender Weise variiert wird. Die Tonhöhe ($L_2$) des anderen Pulszugs bleibt konstant. Format wie in Fig. 3a.

Untervariante 1.2: Ermittlung der CR-Therapietöne mit Zentralton

Hierbei handelt es sich um eine weniger bevorzugte Variante, da bei kleinen durch starkes Entrainment gekennzeichnet Bereichen (welche einem kleinen Herd bzw. kleinen Herden der krankhaften Synchronisation im zentralen Nervensystem entsprechen) in einem ungünstigen Fall neben dem Zentralton kein weiterer Therapieton im Bereich mit starkem Entrainment platziert werden kann. Im Gegensatz hierzu könnten in solch einem Fall ohne Zentralton typischerweise zwei symmetrisch angeordnete Therapietöne platziert werden.

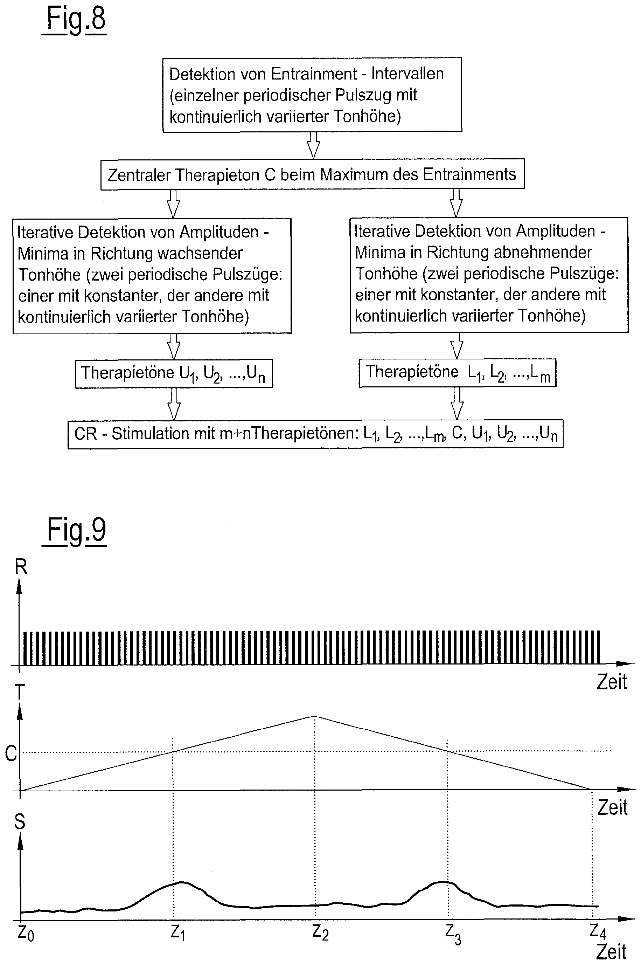

Fig. 8 zeigt ein Flussdiagramm des Ablaufs des beispielhaften Verfahrens bei der Variante mit zentral angeordneten Therapieton (C, sog. Zentralton).

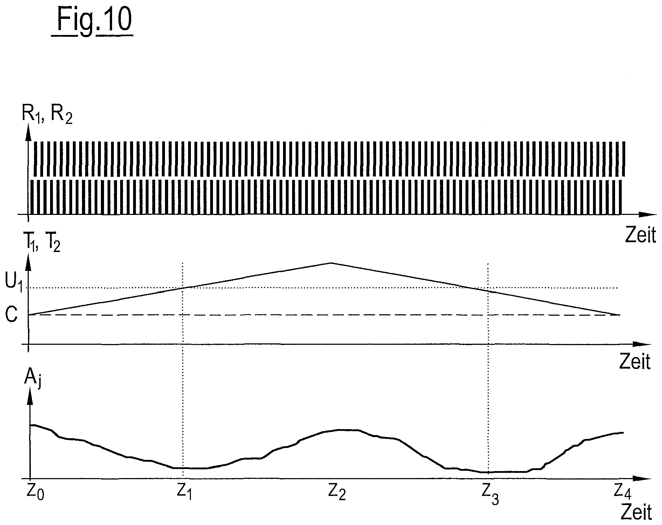

Nach Bestimmung des Zentraltons C wird dieser als Therapieton ausgewählt. Der nächst höher gelegene Therapieton $U_1$ bzw. nächst tiefer gelegene Therapieton $L_1$ werden - analog zur Bestimmung von $U_2$ bzw. $L_2$ im Falle der Variante ohne Zentralton (s.o.) - dadurch detektiert, dass bei den beiden gegenphasigen Reizfolgen die Tonhöhe nur einer Reizfolge anwächst (zur Bestimmung von $U_1$, Fig. 10) bzw. absinkt (zur Bestimmung von $L_1$), während jeweils die Tonhöhe der anderen Reizfolge konstant auf C gesetzt bleibt. Die Bestimmung der jeweiligen Minima bzw. Wendepunkte der Amplitude erfolgt wie oben (für Untervariante I.1) beschrieben. Ebenso erfolgt die weitere iterative Bestimmung der weiteren nächst höher gelegenen Therapietöne $U_2$, $U_3$,... bzw. nächst tiefer gelegenen Therapietöne $L_2$, $L_3$,... wie oben (für Untervariante I.1) beschrieben.

Fig. 9 zeigt die Detektion von Entrainment-Intervallen (einzelner periodischer Pulszug mit kontinuierlich variierter Tonhöhe). Dieser Schritt ist bei den beispielhaften Verfahren mit und ohne Zentralton identisch; dementsprechend sind Fig. 9 und Fig. 2 identisch.

Fig. 10 zeigt die Applikation zweier konstanter periodischer gegenphasiger Pulszüge ($R_1$ und $R_2$), welche aus reinen Tönen mit jeweils der Tonhöhe $T_1$ und $T_2$ bestehen. Die Tonhöhe (durchgezogene Linie) nur eines der beiden Pulszüge wird kontinuierlich in auf- und danach absteigender Weise variiert. Die Tonhöhe (C, gestrichelte Linie) des anderen Pulszugs bleibt konstant. Format wie in Fig. 3a.

**Minimalvarianten:**

Es gibt Ausgestaltungsvarianten der erfindungsgemäßen Vorrichtung und des beispielhaften Verfahrens, bei denen nicht die beiden (in Fig. 1 schematisch dargestellten) Schritte, die Detektion von Entrainment-Intervallen (mittels eines einzelnen periodischen Pulszugs) und die Detektion von Intervallen mit Amplituden-Minima (mittels zweier periodischer Pulszüge), durchgeführt werden, sondern nur einer der beiden Schritte:

**Minimalvariante 1:**

Es wird nur eine Detektion von Entrainment-Intervallen (mittels eines einzelnen periodischen Pulszugs) durchgeführt. An das hierdurch ermittelte Maximum werden z.B. 4 Therapietöne in einem vorgegebenen Frequenz-(d.h. Tonhöhen-)Verhältnis angepasst. Bsp.: Befindet sich das Maximum bei der Tonhöhe C (siehe Fig. 1), können z.B. logarithmisch äquidistante Therapietöne in einem Intervall von z.B. 77% bis 140% von C gewählt werden. Vorteilhaft hierbei ist, dass diese Variante noch deutlich schneller ist als die Variante mit die Variante mit zusätzlicher nachfolgender Detektion von Intervallen mit Amplituden-Minima (mittels zweier periodischer Pulszüge).

**Minimalvariante 2:**

Es wird nur eine Abschätzung der geeigneten Distanz der Therapietöne durchgeführt. Die hierfür nötige Startfrequenz wird vorab gemäß krankheitsspezifischer Kriterien (s.u.) gewählt. Es wird nur eine Detektion von Intervallen mit Amplituden-Minima (mittels zweier periodischer Pulszüge) durchgeführt. Dies ist bei Erkrankungen vorteilhaft, bei denen kein synchroner Fokus im auditorischen Cortex (bzw. im zentralen auditorischen System) in einem mit einem Hörschaden assoziierten Bereich zu erwarten ist, also z.B. bei ADHS, Depression, Schizophrenie etc. Eine Startfrequenz wird z.B. im Sprachbereich festgelegt, was z.B. bei Schizophrenen mit akustischen Halluzinationen vorteilhaft sein kann, das diese im zugehörigen auditorischen Cortex krankhafte Aktivität aufweisen. Diese Startfrequenz entspricht der Tonhöhe C (siehe Fig. 1). Von dem so festgelegten C aus beginnend werden geeignet distante Therapietöne mittels Detektion von Intervallen mit Amplituden-Minima (mittels zweier periodischer Pulszüge) bestimmt. Minimalvariante 2 ist schneller als die Variante mit vorgeschalteter Detektion von Entrainment-Intervallen (mittels eines einzelnen periodischen Pulszugs).

**Auftreten von mehr als einem synchronen Fokus:**

Sollte sich bei der Detektion der Maxima (Fig. 2) mehr als ein Maximum im Teilintervall $[z_0, z_2]$ ergeben, wird der hier beschriebene Algorithmus für jedes lokale Maximum gestartet, bis alle Maxima abgearbeitet und somit alle relevanten Tonhöhen-Teilintervalle abgedeckt sind.

**(II) Diskrete (bzw. grobschrittige) Variation einer (oder mehrerer) Stimuluseigenschaft(en) während der Applikation eines streng periodischen Pulszugs:**

Statt der wie oben beschriebenen kontinuierlichen Variation einer oder mehrerer Stimuluseigenschaften, kann z.B. auch nur für eine diskrete Menge von Werten dieser Stimuluseigenschaft(en) die oben angeführte Entrainment-Analysen (mit einem periodisch applizierten Reiz) bzw. Amplituden-Analysen (mit zwei gegenphasigen periodischen Reizen) durchgeführt werden. Ein derartiges grobschrittiges Vorgehen kann nachteilig sein, da ggfs. umschriebene Hörsenken und damit verbundene umschriebene synchrone Foci nicht detektiert werden können. Um dies zu vermeiden, kann die Schrittweite hinreichend klein gewählt werden. In diesem Fall ist aber die kontinuierliche Variation der Stimuluseigenschaft(en) typischerweise die schnellere Methode. Dies liegt daran, dass bei der diskreten Variation für einen konkreten Wert einer Stimuluseigenschaft hinreichend viele, z.B. N identische Einzelreize in der periodischen Reizfolge appliziert werden müssen, um eine Analyse durchführen zu können (s.o.). Bei der Variante mit kontinuierlicher Variation wird in einem Analyseintervall, das über den Datensatz zeitlich geschoben wird, für einen Intervall von Werten der Stimuluseigenschaft diese N verschiedenen Reize ausgewertet. D.h., in letzterem Fall ist die Auflösung bzgl. der Stimuluseigenschaft(en), z.B. bzgl. der Tonhöhe der Reize (s.o.) verschmiert. Diese Verschmierung ist aber vorteilhaft, da sie eine erhebliche Zeitersparnis mit sich bringt und die therapeutische Stimulationsmethode keine größere Genauigkeit bei der Detektion der synchronen Foci einerseits und der Abschätzung geeigneter Distanzen zwischen den Stimuluseigenschaften (z.B. Tonhöhen) zwischen den Therapietönen erfordert.

**Patentansprüche**

1. Vorrichtung (1) zur Stimulation von Neuronen mit einer krankhaft synchronen und oszillatorischen neuronalen Aktivität, umfassend

   - eine Stimulationseinheit (11) zur Applikation von pulsförmigen Tönen an einen Patienten, wobei die Töne Neuronen des Patienten stimulieren,
   - eine Messeinheit (12) zum Aufnehmen von Messsignalen, die eine neuronale Aktivität der stimulierten Neuronen wiedergeben,
   - eine Steuer- und Analyseeinheit (10) zur Steuerung der Stimulationseinheit (11) und zur Analyse der Messsignale, wobei die Steuer- und Analyseeinheit (10) derart ausgestaltet ist, dass sie
   - die Stimulationseinheit (11) derart ansteuert, dass diese einen ersten Pulszug mit variierter Tonhöhe appliziert,

   **dadurch gekennzeichnet, dass** die Steuer- und Analyseeinheit (10) derart ausgestaltet ist, dass sie

   - anhand der in Reaktion auf die Applikation des ersten Pulszugs aufgenommenen Messsignale mindestens einen ersten Tonhöhenbereich oder mindestens eine erste Tonhöhe (C) auswählt, in welchem oder bei welcher eine Phasensynchronisation zwischen dem Pulszug und der neuronalen Aktivität vorliegt,
   - die Stimulationseinheit (11) derart ansteuert, dass diese zwei phasenverschobene zweite Pulszüge appliziert, wobei die Tonhöhe der beiden zweiten Pulszüge gegenläufig variiert wird und ein Startwert (C) für die Tonhöhe der zweiten Pulszüge in Abhängigkeit von dem mindestens einen ersten Tonhöhenbereich oder der mindestens einen ersten Tonhöhe (C) gewählt wird, und
   - anhand der in Reaktion auf die Applikation der zweiten Pulszüge aufgenommenen Messsignale mindestens einen zweiten Tonhöhenbereich oder mindestens eine zweite Tonhöhe ($U_1$, $L_1$) auswählt, in welchem oder bei welcher die Amplitude der neuronalen Aktivität minimal ist.

2. Vorrichtung (1) nach Anspruch 1, wobei die Phasenverschiebung der beiden zweiten Pulszüge 180° beträgt.

3. Vorrichtung (1) nach Anspruch 1, wobei die Steuer- und Analyseeinheit (10) derart ausgestaltet ist, dass sie anhand der in Reaktion auf die Applikation des ersten Pulszugs aufgenommenen Messsignale eine Tonhöhe (C) auswählt, bei welcher die Phasensynchronisation zwischen dem Pulszug und der neuronalen Aktivität maximal ist.

4. Vorrichtung (1) nach Anspruch 3, wobei die Steuer- und Analyseeinheit (10) die ausgewählte Tonhöhe (C) als Startwert für die Tonhöhe der zweiten Pulszüge verwendet.

5. Vorrichtung (1) nach einem der vorhergehenden Ansprüche, wobei die Steuer- und Analyseeinheit (10) derart ausgestaltet ist, dass sie anhand der in Reaktion auf die Applikation des ersten Pulszugs aufgenommenen Messsignale denjenigen mindestens einen ersten Tonhöhenbereich oder diejenige mindestens eine erste Tonhöhe auswählt, in welchem oder bei welcher eine n:m-Phasendifferenz $\psi_{n,m}(t) = n\varphi_1(t) - m\varphi_2(t)$ maximal ist, wobei $\varphi_1(t)$ die Phase des ersten Pulszugs, $\varphi_2(t)$ die Phase der neuronalen Aktivität und n sowie m ganze Zahlen sind.

6. Vorrichtung (1) nach einem der vorhergehenden Ansprüche, wobei die Steuer- und Analyseeinheit (10) derart ausgestaltet ist, dass sie die Stimulationseinheit (11) derart ansteuert, dass diese zwei phasenverschobene dritte Pulszüge appliziert, wobei die Tonhöhe eines der dritten Pulszüge variiert wird und die Tonhöhe des anderen dritten Pulszugs konstant ist und ein Startwert ($U_1$) für die Tonhöhe der beiden dritten Pulszüge in Abhängigkeit von dem ausgewählten mindestens einen zweiten Tonhöhenbereich oder der ausgewählten mindestens einen zweiten Tonhöhe ($U_1$, $L_1$) gewählt wird, wobei der Startwert ($U_1$) für die Tonhöhe der dritten Pulszüge oberhalb des Startwerts (C) für die Tonhöhe der zweiten Pulszüge liegt und wobei die Steuer- und Analyseeinheit (10) anhand der in Reaktion auf die Applikation der dritten Pulszüge aufgenommenen Messsignale mindestens einen dritten Tonhöhenbereich oder mindestens eine dritte Tonhöhe ($U_2$) auswählt, in welchem oder bei welcher die Amplitude der neuronalen Aktivität minimal ist.

7. Vorrichtung (1) nach Anspruch 6, wobei die Steuer- und Analyseeinheit (10) derart ausgestaltet ist, dass der Schritt nach Anspruch 6 iterativ wiederholt wird.

8. Vorrichtung (1) nach Anspruch 6 oder 7, wobei die Steuer- und Analyseeinheit (10) derart ausgestaltet ist, dass sie die Stimulationseinheit (11) derart ansteuert, dass diese zwei phasenverschobene vierte Pulszüge appliziert, wobei die Tonhöhe eines der vierten Pulszüge variiert wird und die Tonhöhe des anderen vierten Pulszugs konstant ist

und ein Startwert ($L_1$) für die Tonhöhe der beiden vierten Pulszüge in Abhängigkeit von dem ausgewählten mindestens einen zweiten Tonhöhenbereich oder der ausgewählten mindestens einen zweiten Tonhöhe ($U_1$, $L_1$) gewählt wird, wobei der Startwert ($U_1$) für die Tonhöhe der vierten Pulszüge unterhalb des Startwerts ($C$) für die Tonhöhe der zweiten Pulszüge liegt und wobei die Steuer- und Analyseeinheit (10) anhand der in Reaktion auf die Applikation der vierten Pulszüge aufgenommenen Messsignale mindestens einen vierten Tonhöhenbereich oder mindestens eine vierte Tonhöhe ($L_2$) auswählt, in welchem oder bei welcher die Amplitude der neuronalen Aktivität minimal ist.

9. Vorrichtung (1) nach Anspruch 8, wobei die Steuer- und Analyseeinheit (10) derart ausgestaltet ist, dass der Schritt nach Anspruch 8 iterativ wiederholt wird.

10. Vorrichtung (1) nach einem der vorhergehenden Ansprüche, wobei die Steuer- und Analyseeinheit (10) derart ausgestaltet ist, dass sie Töne mit der mindestens einen zweiten Tonhöhe ($U_1$, $L_1$), der mindestens einen dritten Tonhöhe ($U_2$) und der mindestens einen vierten Tonhöhe ($L_2$) für eine therapeutische Stimulation des Patienten auswählt.

11. Vorrichtung (1) nach Anspruch 10, wobei die Steuer- und Analyseeinheit (10) derart ausgestaltet ist, dass sie zusätzlich einen Ton mit der mindestens einen ersten Tonhöhe ($C$) für die therapeutische Stimulation des Patienten auswählt.

12. Vorrichtung (1) nach Anspruch 10 oder 11, wobei die therapeutische Stimulation eine "Coordinated Reset"-Stimulation ist.

13. Vorrichtung (1) zur Stimulation von Neuronen mit einer krankhaft synchronen und oszillatorischen neuronalen Aktivität, umfassend

- eine Stimulationseinheit (11) zur Applikation von pulsförmigen Tönen an einen Patienten, wobei die Töne Neuronen des Patienten stimulieren,
- eine Messeinheit (12) zum Aufnehmen von Messsignalen, die eine neuronale Aktivität der stimulierten Neuronen wiedergeben,
- eine Steuer- und Analyseeinheit (10) zur Steuerung der Stimulationseinheit (11) und zur Analyse der Messsignale, wobei die Steuer- und Analyseeinheit (10) derart ausgestaltet ist, dass sie
- die Stimulationseinheit (11) derart ansteuert, dass diese einen ersten Pulszug mit variierter Tonhöhe appliziert,

**dadurch gekennzeichnet, dass** die Steuer- und Analyseeinheit (10) derart ausgestaltet ist, dass sie

- anhand der in Reaktion auf die Applikation des ersten Pulszugs aufgenommenen Messsignale mindestens einen ersten Tonhöhenbereich oder mindestens eine erste Tonhöhe ($C$) auswählt, in welchem oder bei welcher eine Phasensynchronisation zwischen dem Pulszug und der neuronalen Aktivität vorliegt,
- die Stimulationseinheit (11) derart ansteuert, dass diese zwei phasenverschobene zweite Pulszüge appliziert, wobei die Tonhöhe eines der zweiten Pulszüge variiert wird und die Tonhöhe des anderen zweiten Pulszugs konstant ist und ein Startwert ($C$) für die Tonhöhe der zweiten Pulszüge in Abhängigkeit von dem mindestens einen ersten Tonhöhenbereich oder der mindestens einen ersten Tonhöhe ($C$) gewählt wird, und
- anhand der in Reaktion auf die Applikation der zweiten Pulszüge aufgenommenen Messsignale mindestens einen zweiten Tonhöhenbereich oder mindestens eine zweite Tonhöhe ($U_1$, $L_1$) auswählt, in welchem oder bei welcher die Amplitude der neuronalen Aktivität minimal ist.

**Claims**

1. Apparatus (1) for stimulating neurons having a pathologically synchronous and oscillatory neural activity, comprising

- a stimulation unit (11) for the application of pulse-like sounds to a patient, wherein the sounds stimulate neurons of the patient;
- a measuring unit (12) for recording measured signals which reproduce a neural activity of the stimulated neurons;
- a control and analysis unit (10) for controlling the stimulation unit (11) and for analyzing the measured signals, wherein the control and analysis unit (10) is configured such that it
- controls the stimulation unit (11) such that it applies a first pulse train with a varied pitch,

**characterized in that** the control and analysis unit (10) is configured such that it;

- selects at least one first pitch range or at least one first pitch (C) in or at which a phase synchronization is present between the pulse train and the neural activity with reference to the measured signals recorded in response to the application of the first pulse train;
- controls the stimulation unit (11) such that it applies two phase-shifted second pulse trains, wherein the pitch of the two second pulse trains is varied oppositely and a start value (C) is selected for the pitch of the second pulse trains in dependence on the at least one first pitch range or on the at least one first pitch (C); and
- selects at least one second pitch range or at least one second pitch (U₁, L₁) in or at which the amplitude of the neural activity is minimal with reference to the measured signals recorded in response to the application of the second pulse trains.

2. Apparatus (1) in accordance with claim 1, wherein the phase shift of the two second pulse trains amounts to 180°.

3. Apparatus (1) in accordance with claim 1, wherein the control and analysis unit (10) is configured such that it selects a pitch (C) at which the phase synchronization between the pulse train and the neural activity is a maximum with reference to the measured signals recorded in response to the application of the first pulse train.

4. Apparatus (1) in accordance with claim 3, wherein the control and analysis unit (10) uses the selected pitch (C) as a start value for the pitch of the second pulse trains.

5. Apparatus (1) in accordance with any one of the preceding claims, wherein the control and analysis unit (10) is configured such that it selects that at least one first pitch range or that at least one first pitch in or at which an n:m phase difference $\psi_{n,m}(t) = n\varphi_1(t) - m\varphi_2(t)$ is a maximum with reference to the measured signals recorded in response to the application of the first pulse train, wherein $\varphi_1(t)$ is the phase of the first pulse train, $\varphi_2(t)$ is the phase of neural activity and n and m are whole numbers.

6. Apparatus (1) in accordance with any one of the preceding claims, wherein the control and analysis unit (10) is configured such that it controls the stimulation unit (11) such that it applies two phase-shifted third pulse trains, wherein the pitch of one of the third pulse trains is varied and the pitch of the other third pulse train is constant and a start value (U₁) for the pitch of the two third pulse trains is selected in dependence on the selected at least one second pitch range or on the selected at least one second pitch (U₁, L₁), wherein the start value (U₁) for the pitch of the third pulse trains is above the start value (C) for the pitch of the second pulse trains, and wherein the control and analysis unit (10) selects at least one third pitch range or at least one third pitch (U₂) in or at which the amplitude of the neural activity is minimal with reference to the measured signals recorded in response to the application of the third pulse trains.

7. Apparatus (1) in accordance with claim 6, wherein the control and analysis unit (10) is configured such that the step in accordance with claim 6 is iteratively repeated.

8. Apparatus (1) in accordance with claim 6 or claim 7, wherein the control and analysis unit (6) is configured such that it controls the stimulation unit (11) such that it applies two phase-shifted fourth pulse trains, wherein the pitch of one of the fourth pulse trains is varied and the pitch of the other fourth pulse train is constant and a start value (L₁) for the pitch of the two fourth pulse trains is selected in dependence on the selected at least one second pitch range or on the selected at least one second pitch (U₁, L₁), wherein the start value (U₁) for the pitch of the fourth pulse trains is below the start value (C) for the pitch of the second pulse trains, and wherein the control and analysis unit (10) selects at least one fourth pitch range or at least one fourth pitch (L₂) in or at which the amplitude of the neural activity is minimal with reference to the measured signals recorded in response to the application of the fourth pulse trains.

9. Apparatus (1) in accordance with claim 8, wherein the control and analysis unit (10) is configured such that the step in accordance with claim 8 is iteratively repeated.

10. Apparatus (1) in accordance with any one of the preceding claims, wherein the control and analysis unit (10) is configured such that it selects sounds having the at least one second pitch (U₁, L₁), having the at least one third pitch (U₂) and having the at least one fourth pitch (L₂) for a therapeutic stimulation of the patient.

11. Apparatus (1) in accordance with claim 10, wherein the control and analysis unit (10) is configured such that it additionally selects a sound having the at least one first pitch (C) for the therapeutic stimulation of the patient.

12. Apparatus (1) in accordance with claim 10 or claim 11, wherein the therapeutic stimulation is a "coordinated reset" stimulation.

13. Apparatus (1) for stimulating neurons having a pathologically synchronous and oscillatory neural activity, comprising

    - a stimulation unit (11) for the application of pulse-like sounds to a patient, wherein the sounds stimulate neurons of the patient;
    - a measuring unit (12) for recording measured signals which reproduce a neural activity of the stimulated neurons;
    - a control and analysis unit (10) for controlling the stimulation unit (11) and for analyzing the measured signals, wherein the control and analysis unit (10) is configured such that it
    - controls the stimulation unit (11) such that it applies a first pulse train with a varied pitch,

    **characterized in that** the control and analysis unit (10) is configured such that it;

    - selects at least one first pitch range or at least one first pitch (C) in or at which a phase synchronization is present between the pulse train and the neural activity with reference to the measured signals recorded in response to the application of the first pulse train;
    - controls the stimulation unit (11) such that it applies two phase-shifted pulse trains, wherein the pitch of one of the second pulse trains is varied and the pitch of the other second pulse train is constant and a start value (C) is selected for the pitch of the second pulse trains in dependence on the at least one first pitch range or on the at least one first pitch (C); and
    - selects at least one second pitch range or at least one second pitch ($U_1$, $L_1$) in or at which the amplitude of the neural activity is minimal with reference to the measured signals recorded in response to the application of the second pulse trains.

**Revendications**

1. Dispositif (1) pour stimuler des neurones ayant une activité neuronale pathologiquement synchrone et oscillatoire comprenant:

    - une unité de stimulation (11) pour appliquer des sons pulsées à un patient, les sons stimulant des neurones du patient,
    - une unité de mesure (12) pour enregistrer des signaux de mesure reflétant une activité neuronale des neurones stimulés,
    - une unité de commande et d'analyse (10) pour commander l'unité de stimulation (11) et pour analyser les signaux de mesure, sachant que l'unité de commande et d'analyse (10) est configurée de telle façon qu'elle
    - commande l'unité de stimulation (11) de manière qu'elle applique un premier train d'impulsions à hauteur de son variable,

    **caractérisée en ce que** l'unité de commande et d'analyse (10) est conçue de manière qu'elle

    - sélectionne, sur la base des signaux de mesure enregistrés en réponse à une application d'un premier train d'impulsions, au moins une première gamme de hauteurs de son ou au moins une première hauteur de son (C), dans laquelle ou à laquelle se présente une synchronisation de phase entre le train d'impulsions et l'activité neuronale,
    - commande l'unité de stimulation (11) de telle manière que celle-ci applique deux trains d'impulsions secondaires décalés de phase, sachant que la hauteur de son des deux trains d'impulsions secondaires est variée dans des directions opposées et qu'une valeur de départ (C) est sélectionnée pour la hauteur de son des deux trains d'impulsions secondaires en fonction d'au moins une première gamme de hauteurs de son ou au moins de hauteur de son initiale (C), et qu'elle
    - sélectionne sur la base des signaux de mesure enregistrés en réponse à l'application des trains d'impulsions secondaires, au moins une deuxième gamme de hauteurs de son ou au moins une deuxième hauteur de son ($U_1$, $L_1$), dans laquelle ou à laquelle l'amplitude de l'activité neuronale est minimale.

**2.** Dispositif (1) d'après la revendication 1, sachant que le déphasage des deux trains d'impulsions secondaires est de 180°.

**3.** Dispositif (1) d'après la revendication 1, sachant que l'unité de commande et d'analyse (10) est configurée de manière qu'elle sélectionne une hauteur de son (C) sur la base des signaux de mesure enregistrés en réponse à l'application du premier train d'impulsions, à laquelle la synchronisation de phase entre le train d'impulsions et l'activité neuronale est maximale.

**4.** Dispositif (1) d'après la revendication 3, sachant que l'unité de commande et d'analyse (10) utilise la hauteur de son (C) sélectionnée comme valeur de départ pour la hauteur de son des deuxièmes trains d'impulsions.

**5.** Dispositif (1) d'après une des revendications précédentes, sachant que l'unité de commande et d'analyse (10) est conçue de manière qu'elle sélectionne, sur la base des signaux de mesure enregistrés en réponse à l'application du premier train d'impulsions, au moins la première gamme de hauteurs de son ou au moins la première hauteur de son dans laquelle ou à laquelle une différence de phase n:m $\psi_{n,m}(t) = n\varphi_1(t) - m\varphi_2(t)$ est maximale, sachant que $\varphi_1(t)$ est la phase du premier train d'impulsions, $\varphi_2(t)$ est la phase de l'activité neuronale et n et m sont des nombres entiers.

**6.** Dispositif (1) d'après une des revendications précédentes, sachant que l'unité de commande et d'analyse (10) est conçue de manière qu'elle commande l'unité de stimulation (11) de telle manière qu'elle applique deux trains d'impulsions tiers décalés de phase, sachant que la hauteur de son d'un des troisièmes trains d'impulsions est variée, et que la hauteur de son de l'autre troisième train d'impulsions est constante, et qu'une valeur de départ $(U_1)$ pour la hauteur de son des deux troisièmes trains d'impulsions est sélectionnée en fonction de la ou des deuxièmes gammes de hauteurs de son sélectionnées ou de la ou des deuxièmes hauteurs de son sélectionnées $(U_1, L_1)$, sachant que la valeur de départ $(U_1)$ pour la hauteur de son des troisièmes trains d'impulsions est supérieure à la valeur de départ (C) pour la hauteur de son des deuxièmes trains d'impulsions, et sachant que l'unité de commande et d'analyse (10) sélectionne, sur la base des signaux de mesure enregistrés en réponse à l'application des troisièmes trains d'impulsions, au moins une troisième gamme de hauteurs de son ou au moins une troisième hauteur de son $(U_2)$, dans laquelle ou à laquelle l'amplitude de l'activité neuronale est minimale.

**7.** Dispositif (1) d'après la revendication 6, sachant que l'unité de commande et d'analyse (10) est conçue de manière que l'étape selon la revendication 6 est répétée itérativement.

**8.** Dispositif (1) d'après la revendication 6 ou 7, sachant que l'unité de commande et d'analyse (10) est conçue de manière qu'elle commande l'unité de stimulation (11) de telle façon que celle-ci applique deux quatrièmes trains d'impulsions décalés de phase, sachant que la hauteur de son de l'un des quatrièmes trains d'impulsions est variée, et que la hauteur de son de l'autre quatrième train d'impulsions est constante, et qu'une valeur de départ $(L_1)$ pour la hauteur de son des deux quatrièmes trains d'impulsions est sélectionnée en fonction de la ou des secondes gammes de hauteurs de son sélectionnées ou en fonction de la ou des secondes hauteurs de son sélectionnées $(U_1, L_1)$, sachant que la valeur de départ $(U_1)$ pour la hauteur de son des quatrième trains d'impulsions est inférieure à la valeur de départ (C) pour la hauteur de son des deuxième trains d'impulsions, et sachant que l'unité de commande et d'analyse (10) sélectionne, sur la base des signaux de mesure enregistrés en réponse à l'application des quatrième trains d'impulsions, au moins une quatrième gamme de hauteurs de son ou au moins une quatrième hauteur de son $(L_2)$, dans laquelle ou à laquelle l'amplitude de l'activité neuronale est minimale.

**9.** Dispositif (1) d'après la revendication 8, sachant que l'unité de commande et d'analyse (10) est conçue de manière que l'étape selon la revendication 8 est répétée itérativement.

**10.** Dispositif (1) d'après une des revendications précédentes, sachant que l'unité de commande et d'analyse (10) est conçue de manière qu'elle sélectionne des sons ayant la ou les deuxièmes hauteurs de son $(U_1, L_1)$, la ou les troisièmes hauteurs de son $(U_2)$ et la ou les quatrième hauteurs de son $(L_2)$ pour une stimulation thérapeutique du patient.

**11.** Dispositif (1) d'après la revendication 10, sachant que l'unité de commande et d'analyse (10) est conçue de manière qu'elle sélectionne en outre un son ayant la ou les premières hauteurs de son (C) pour la stimulation thérapeutique du patient.

**12.** Dispositif (1) d'après la revendication 10 ou 11, sachant que la stimulation thérapeutique est une stimulation du type «*Coordinated Reset* ».

13. Dispositif (1) pour stimuler des neurones ayant une activité neuronale pathologiquement synchrone et oscillatoire comprenant:

- une unité de stimulation (11) pour appliquer des sons pulsées à un patient, les sons stimulant des neurones du patient,
- une unité de mesure (12) pour enregistrer des signaux de mesure reflétant une activité neuronale des neurones stimulés,
- une unité de commande et d'analyse (10) pour commander l'unité de stimulation (11) et pour analyser les signaux de mesure, sachant que l'unité de commande et d'analyse (10) est configurée de telle façon qu'elle
- commande l'unité de stimulation (11) de manière qu'elle applique un premier train d'impulsions à hauteur de son variable,

**caractérisée en ce que** l'unité de commande et d'analyse (10) est conçue de manière qu'elle

- sélectionne, sur la base des signaux de mesure enregistrés en réponse à une application d'un premier train d'impulsions, au moins une première gamme de hauteurs de son ou au moins une première hauteur de son (C), dans laquelle ou à laquelle se présente une synchronisation de phase entre le train d'impulsions et l'activité neuronale,
- commande l'unité de stimulation (11) de telle manière que celle-ci applique deux trains d'impulsions secondaires décalés de phase, sachant que la hauteur de son d'un des secondaires trains d'impulsions est variée et que la hauteur de son de l'autre secondaire train d'impulsions est constante et qu'une valeur de départ (C) est sélectionnée pour la hauteur de son des deux trains d'impulsions secondaires en fonction d'au moins une première gamme de hauteurs de son ou au moins de hauteur de son initiale (C), et qu'elle
- sélectionne sur la base des signaux de mesure enregistrés en réponse à l'application des trains d'impulsions secondaires, au moins une deuxième gamme de hauteurs de son ou au moins une deuxième hauteur de son ($U_1$, $L_1$), dans laquelle ou à laquelle l'amplitude de l'activité neuronale est minimale.

## Fig.1

| Detektion von Entrainment - Intervallen (einzelner periodischer Pulszug mit kontinuierlich variierter Tonhöhe) |

⇩

| Detektion von Intervallen mit Amplituden - Minima (zwei periodische Pulszüge mit kontinuierlich variierter Tonhöhe) |

⇩                                        ⇩

| Iterative Detektion von Amplituden - Minima in Richtung wachsender Tonhöhe (zwei periodische Pulszüge: einer mit konstanter, der andere mit kontinuierlich variierter Tonhöhe) | | Iterative Detektion von Amplituden - Minima in Richtung abnehmender Tonhöhe (zwei periodische Pulszüge: einer mit konstanter, der andere mit kontinuierlich variierter Tonhöhe) |

⇩                                        ⇩

| Therapietöne $U_1$, $U_2$, ...,$U_n$ | | Therapietöne $L_1$, $L_2$, ...,$L_m$ |

⇩                                        ⇩

| CR - Stimulation mit m+nTherapietönen: $L_1$, $L_2$, ...,$L_m$, $U_1$, $U_2$, ...,$U_n$ |

## Fig.2

## Fig.3a

## Fig.3b

## Fig.4

## Fig.5

EP 3 010 572 B1

## Fig.6

R₁, R₂

Zeit

T₁, T₂

L₁
L₂

Zeit

Aⱼ

Z₀    Z₁    Z₂    Z₃    Z₄

Zeit

## Fig.7

R₁, R₂

Zeit

T₁, T₂

L₂
L₃

Zeit

Aⱼ

Z₀    Z₁    Z₂    Z₃    Z₄

Zeit

24

## Fig.8

Detektion von Entrainment - Intervallen
(einzelner periodischer Pulszug mit
kontinuierlich variierter Tonhöhe)

⇩

Zentraler Therapieton C beim Maximum des Entrainments

⇩ ⇩

Iterative Detektion von Amplituden -
Minima in Richtung wachsender
Tonhöhe (zwei periodische Pulszüge:
einer mit konstanter, der andere mit
kontinuierlich variierter Tonhöhe)

Iterative Detektion von Amplituden -
Minima in Richtung abnehmender
Tonhöhe (zwei periodische Pulszüge:
einer mit konstanter, der andere mit
kontinuierlich variierter Tonhöhe)

⇩ ⇩

Therapietöne $U_1, U_2, ..., U_n$

Therapietöne $L_1, L_2, ..., L_m$

⇩ ⇩

CR - Stimulation mit m+nTherapietönen: $L_1, L_2, ..., L_m, C, U_1, U_2, ..., U_n$

## Fig.9

Fig.10

## Fig.11

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102010016461 A1

**In der Beschreibung aufgeführte Nicht-Patentliteratur**

- **P.A. TASS ; I. ADAMCHIC ; H.-J. FREUND ; T. VON STACKELBERG ; C.HAUPTMANN.** Counteracting tinnitus by acoustic coordinated reset neuromodulation. *Restorative Neurology and Neuroscience,* 2012, vol. 30, 137-159
- **I. ADAMCHIC ; T. TOTH ; C. HAUPTMANN ; P.A. TASS.** Reversing pathologically increased EEG power by acoustic CR neuromodulation. *Human Brain Mapping*
- **A.N. SILCHENKO ; I. ADAMCHIC ; C. HAUPTMANN ; P.A. TASS.** Impact of acoustic coordinated reset neuromodulation on effective connectivity in a neural network of phantom sound. *Neuroimage,* 2013, vol. 77, 133-147
- Phase Synchronization: From Theory to Data Analysis. **M.G. ROSENBLUM ; A.S. PIKOVSKY ; C. SCHÄFER ; J. KURTHS ; P.A. TASS.** Handbook of Biological Physics. Elsevier, 2000
- **HUANG N.E. et al.** The empirical mode decomposition and the Hilbert spectrum for nonlinear and non-stationary time series analysis. *Proc R Soc A: Math Phys Eng Sci,* 1998, vol. 454, 903-995
- **AU HUANG, NE et al.** A confidence limit for the empirical mode decomposition and Hilbert spectral analysis. *Proceedings of the Royal Society of London Series A,* 2003, vol. 459, 2317-2345
- **P. TASS ; M.G. ROSENBLUM ; J. WEULE ; J. KURTHS ; A. PIKOVSKY ; J. VOLKMANN ; A. SCHNITZLER ; H.-J. FREUND.** Detection of n:m Phase Locking from Noisy Data: Application to Magnetoencephalography. *Phys. Rev. Lett.,* 1998, vol. 81 (15), 3291-3294
- **E. BATSCHELET.** Circular Statistics in Biology. Academic Press, 1981
- Tests concerning random points in a circle. **N. H. KUIPER.** Proc. K. Ned. Akad. Wet., Ser. A: Math. Sci. 1960, vol. 63, 38
- **P.A. TASS.** Transmission of stimulus-locked responses in two coupled phase oscillators. *Phys. Rev. E,* 2004, vol. 69, 051909-1, 24
- **P. LANDA.** Nonlinear Oscillations and Waves in Dynamical Systems. Kluwer Academic Publishers, 1996
- **TAYLOR, WAYNE.** Change-Point Analyzer 2.0 shareware program. Taylor Enterprises, 2000
- **BÉKÉSY G. V.** A new audiometer. *Acta oto-laryngol,* 1947, vol. 35, 411
- **LEHNHARDT E. ; LASZIG R.** Praxis der Audiometrie. Thieme, 2009